# FASCICULE DE BREVET EUROPEEN

(11) **EP 2 920 594 B1**
(45) Date de publication et mention de la délivrance du brevet: **10.01.2018**
(21) Numéro de dépôt: 13815035.4
(22) Date de dépôt: 19.11.2013
(51) Int. Cl.: C07D 213/20, C07D 213/65, G01N 33/50, G01N 33/68, A61K 49/00

(54) **MODÈLE CHIMIQUE D'UNE MALADIE NEURODÉGÉNÉRATIVE, PROCÉDÉ DE PRÉPARATION ET UTILISATIONS**
CHEMISCHES MODELL EINER NEURODEGENERATIVEN ERKRANKUNG, VERFAHREN ZUR HERSTELLUNG UND VERWENDUNGEN DAVON
CHEMICAL MODEL OF A NEURODEGENERATIVE DISEASE, METHOD FOR PREPARATION AND USES OF SAME

(30) Priorité: 19.11.2012 FR 1260979
(43) Date de publication de la demande: 23.09.2015
(73) Titulaire: Centre National de la Recherche Scientifique, 75016 Paris (FR); Université Paris-Est Créteil Val de Marne, 94010 Creteil Cedex (FR)
(72) Inventeur: MARTENS, Thierry, F-94510 La Queue En Brie (FR); RIVARD, Michaël, F-94000 Creteil (FR); LAURENCÉ, Céline, F-94190 Villeneuve Saint Georges (FR); MORIN, Christophe, F-94100 Saint Maur Des Fosses (FR); LEHRI-BOUFALA, Sonia, 94700 Maisons-Alfort (FR); ZEGHBIB, Narimane, 94460 Valenton (FR)
(74) Mandataire: Regimbeau
(86) Numéro de dépôt international: PCT/FR2013/052784
(87) Numéro de publication internationale: WO 2014/076439

(56) Documents cités:
- GB-A- 1 544 688
- ZHAO S ET AL: "An efficient ultrasonic-assisted synthesis of imidazolium and pyridinium salts based on the Zincke reaction", ULTRASONICS: SONOCHEMISTRY, BUTTERWORTH-HEINEMANN, GB, vol. 17, no. 4, 1 avril 2010 (2010-04-01), pages 685-689, XP026949390, ISSN: 1350-4177, DOI: 10.1016/J.ULTSONCH.2009.12.019 [extrait le 2010-01-04]
- WOJTAS L ET AL: "Conformation and interactions of 4-(pyridinium-1-yl)-phenolate betaine-dye and its cation in the crystalline state", JOURNAL OF MOLECULAR STRUCTURE, ELSEVIER, AMSTERDAM, NL, vol. 785, no. 1-3, 6 mars 2006 (2006-03-06), pages 14-20, XP028045901, ISSN: 0022-2860, DOI: 10.1016/J.MOLSTRUC.2005.09.023 [extrait le 2006-03-06]
- SUSAN DUTY ET AL: "Animal models of Parkinson's disease: a source of novel treatments and clues to the cause of the disease", BRITISH JOURNAL OF PHARMACOLOGY, vol. 164, no. 4, 1 octobre 2011 (2011-10-01), pages 1357-1391, XP055078955, ISSN: 0007-1188, DOI: 10.1111/j.1476-5381.2011.01426.x
- JAVIER BLESA ET AL: "Classic and New Animal Models of Parkinson's Disease", JOURNAL OF BIOMEDICINE AND BIOTECHNOLOGY, vol. 25, no. 1, 1 janvier 2012 (2012-01-01), pages 239-10, XP055078789, ISSN: 1110-7243, DOI: 10.1111/j.1471-4159.2010.07036.x
- BETARBET R ET AL: "ANIMAL MODELS OF PARKINSON'S DISEASE", BIOESSAYS, JOHN WILEY & SONS LTD, GB, vol. 24, no. 4, 1 janvier 2002 (2002-01-01), pages 308-318, XP002547553, ISSN: 0265-9247, DOI: 10.1002/BIES.10067
- BLANDINI FABIO ET AL: "Animal models of Parkinson's disease", FEBS JOURNAL, vol. 279, no. 7, avril 2012 (2012-04), pages 1156-1166, XP002712776,
- LING-JEN CHEN ET AL: "Chemical and Enzymatic Oxidation of Furosemide: Formation of Pyridinium Salts", CHEMICAL RESEARCH IN TOXICOLOGY, vol. 20, no. 12, 1 décembre 2007 (2007-12-01), pages 1741-1744, XP055100824, ISSN: 0893-228X, DOI: 10.1021/tx700262z
- Yoshito Takeuchi ET AL: "I3C NMR Spectra of Pyridinium Betaines and Related Compounds", , 1 January 1976 (1976-01-01), page 27, XP055317703, Retrieved from the Internet: URL:http://onlinelibrary.wiley.com/store/1 0.1002/mrc.1270080107/asset/1270080107_ftp .pdf?v=1&t=ivarrnor&s=ec5b01b544dc3959a13f 77c8ff7c9592250b8364

## Description

### Domaine de l'invention

L'invention concerne la mise au point et l'utilisation de modèles chimiques pour l'étude d'une maladie neurodégénérative, en particulier de la maladie de Parkinson.

### Arrière-plan technologique

La maladie de Parkinson est la seconde maladie neurodégénérative la plus fréquente après la maladie d'Alzheimer. Sa prévalence augmente avec l'âge et atteint 1% à 2% des sujets âgés de plus de 50 ans. Elle est caractérisée par des symptômes moteurs invalidants tels qu'une akinésie, une rigidité musculaire et des tremblements au repos. Ces manifestations cliniques peuvent être accompagnées de troubles non-moteurs tels que des troubles digestifs, urinaires ou cognitifs.

Les troubles moteurs associés à la maladie de Parkinson sont directement liés à la dégénérescence des neurones dopaminergiques de la *substantia nigra.*

Les phénomènes moléculaires à la base de cette dégénérescence neuronale n'ont pas été entièrement élucidés mais il est avancé que le stress oxydant, le dysfonctionnement mitochondrial et l'agrégation anormale de protéines jouent un rôle clé dans la pathogénèse de la maladie. L'examen histologique post-mortem d'échantillons provenant de patients atteints par la maladie de Parkinson a mis en évidence, de manière quasi-systématique, l'accumulation et l'agrégation d'une protéine, l'alpha synucléine, au sein d'inclusions ésosinophiles d'aspect fibrillaire, les corps de Lewy (Spillantini, et al., PNAS, 1998, 95, 669-6473). Ces inclusions ont été principalement détectées dans le cytoplasme des neurones dopaminergiques de la *substantia nigra.* De manière remarquable, les corps de Lewy sont observables même en l'absence de mutations du gène de l'alpha-synucléine et sont ainsi considérés comme un marqueur clé de la maladie de Parkinson. Les corps de Lewy ont été également détectés dans des maladies impliquant une perte de neurones dopaminergiques cérébraux comme la démence à corps de Lewy.

Les causes exactes de la maladie de Parkinson restent inconnues. Des facteurs environnementaux - telle que l'exposition aux pesticides ou aux métaux lourds - ont été avancés. En parallèle, l'étude des formes familiales de la maladie de Parkinson a permis d'identifier plusieurs gènes qui seraient impliqués dans l'apparition de la maladie.

Néanmoins, dans la grande majorité des cas, la maladie de Parkinson est sporadique, ce qui suggère une étiologie multifactorielle impliquant des prédispositions génétiques et l'exposition à des facteurs environnementaux.

Il n'existe aucun traitement permettant de guérir de la maladie de Parkinson ou d'en ralentir de manière significative la progression, en particulier la dégénérescence neuronale (Seidl, Front Neurol, 2011, 2, 68). Les seuls traitements disponibles sur le marché ont pour but de réduire les symptômes associés à cette pathologie, tels que les tremblements.

Dans ce contexte, la mise au point de modèles cellulaires et animaux demeurent essentielle pour élucider les mécanismes impliqués dans la maladie de Parkinson et développer des traitements curatifs et/ou préventifs de cette maladie. On pourra se référer à l'article de Betarbet et al., (Bioassays, 2002, 24 :308-318), à l'article de Blesa et al. (Journal of Biomedecine and Biotechnology, 2012, Article ID 845618) ou encore à celui de Blandini et al., (FEBS Journal, 2012, 279, 1156-1166) pour une synthèse concernant les modèles animaux de la maladie de Parkinson.

Ces publications montrent que la modélisation de la maladie de Parkinson comprend généralement l'induction d'une lésion dopaminergique au niveau de la *subtantia nigra* soit par voie chimique, soir par voie génétique.

L'approche chimique consiste à induire certaines caractéristiques biologiques essentielles de la maladie de Parkinson par exposition de l'animal à un agent chimique. La 6-hydroxydopamine (6-OHDA) a été le premier agent chimique utilisé pour mimer la maladie de Parkinson. Néanmoins, ce modèle présente de nombreux inconvénients: en particulier, la 6-OHDA doit être administrée par voie intra-cranienne et présente une toxicité aigue. Par ailleurs, l'administration de 6-OHDA ne provoque ni l'apparition de lésions dans l'ensemble des régions cérébrales impliquées dans la maladie de Parkinson, ni la formation de corps de Lewy. Le modèle 6-OHDA a été supplanté par le modèle MPTP (1-méthyl-4-phényl-1,2,3,6-tétrahydropyridine)/MPP+ qui est, à l'heure actuelle, le modèle le mieux caractérisé et le plus utilisé. Après administration, le MPTP traverse la barrière hémato-encéphalique et est métabolisé par les monoamines oxydases des astrocytes en MPP+ (1-méthyl-4-phényl-2,3-dihydropyridinum), un inhibiteur du complexe I mitochondriale. Le MPP+ cible spécifiquement les neurones dopaminergiques nigrostriaux. L'exposition au MPTP entraine chez de nombreuses espèces, y compris chez l'homme, une dégénérescence des neurones dopaminergiques de la *substantia nigra* et induit certains des symptômes associés à la maladie de Parkinson. Néanmoins, ce modèle présente de nombreuses limitations. Il est généralement très difficile de maintenir en vie les animaux présentant des lésions bilatérales induites par le MPTP sans administration de L-Dopa ou d'agonistes de la dopamine. Par ailleurs, ce modèle ne reproduit pas simultanément l'ensemble des troubles moteurs associés à la maladie de Parkinson. Bien que la présence d'agrégats d'alpha-synucléine sous forme d'inclusions ait été rapportée chez certaines espèces de primates, la formation de corps de Lewy n'a été, en revanche, jamais observée à ce jour (Blesa *et al., supra*). Enfin, le modèle MPTP/MPP+ est un modèle dit « aigu », qui ne reproduit pas la progressivité lente de la maladie de Parkinson, ce qui rend difficile son utilisation pour la découverte de nouvelles solutions thérapeutiques ou l'élucidation de l'ensemble des mécanismes biologiques impliqués dans l'apparition de la maladie de Parkinson.

Plus récemment, d'autres modèles chimiques basés sur un herbicide de type pyridinium, le paraquat, ou d'autres inhibiteurs du complexe I mitochondrial ont été évalués. Ces modèles présentent des limitations analogues à celles du MPTP et notamment une absence de spécificité vis-à-vis du système dopaminergique.

Des modèles génétiques basés sur l'extinction, la mutation ou la surexpression d'un gène d'intérêt tel que l'alpha-synucléine ou LRRK2 (leucine rich repeat kinase 2) ont été également développés. Bien qu'étant des outils d'étude intéressants, ils ne reproduisent pas de manière totalement fidèle la maladie, en particulier l'apparition de corps de Lewy (Dawson et al., Neuron. 2010,66(5):646-61). Par ailleurs, la présence d'une mutation génétique dès la naissance, et même dès l'embryogénèse, chez l'animal peut impliquer de nombreux processus et induire des phénomènes de compensation et d'adaptation qui ne seraient pas observables chez l'individu adulte, ce qui limite et complique la transposition des résultats obtenus à l'homme.

Aucun des modèles chimiques ou génétiques développés jusqu'à présent n'est capable de reproduire l'ensemble des symptômes et les principaux marqueurs de la maladie de Parkinson, en particulier sa progressivité de développement et l'apparition des corps de Lewy.

Il existe donc à l'heure actuelle un besoin de modèles animaux et cellulaires de la maladie de Parkinson alternatifs à ceux décrits dans l'art antérieur.

### Résumé de l'invention

L'invention a pour objet l'utilisation d'un composé de formule (I) 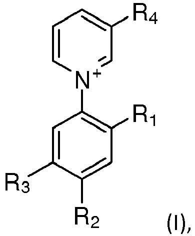 dans laquelle,
- R₁ représente un atome d'hydrogène, un atome d'halogène, un alkyle en C₁-C₄,-OH,-CN, -C(=O)NH₂, -CF₃, -C(=O)O⁻, ou -C(=O)O-R₅ avec R₅ représente H ou un alkyle en C₁-C₄,
- R₂ est -SO₂NH₂, un atome d'hydrogène, un atome d'halogène, un alkyle en C₁-C₄,-OH, -CN, -C(=O)NH₂, -CF₃, ou -C(=O)OR₆ avec R₆ représente un alkyle en C₁-C₄ ou -H,
- R₃ est H, un atome d'halogène, un alkyle en C₁-C₄,-OH,-CN,-C(=O)NH₂,-CF₃, ou - C(=O)OR₇ avec R7 représente -H ou un alkyle en C₁-C₄, et -R₄ représente un atome d'hydrogène, -OH ou -OCH₃,
ou un sel acceptable sur le plan pharmaceutique de celui-ci, dans la préparation d'un modèle chimique d'une synucléinopathie, de préférence de la maladie de Parkinson. Le modèle chimique est obtenu par mise en contact d'un système cellulaire avec ledit composé, ledit système cellulaire étant choisi parmi une cellule, une culture cellulaire, un tissu et un animal non-humain.

Un objet supplémentaire de l'invention est une méthode de préparation d'un modèle chimique d'une synucléinopathie, de préférence de la maladie de Parkinson, comprenant une étape de mise en contact d'un système cellulaire choisi parmi une cellule, une culture cellulaire, un tissu et un animal non-humain avec un composé choisi parmi les composés de formule (I) tels que définis ci-avant, les sels acceptables sur le plan pharmaceutique de ceux-ci et les combinaisons de ceux-ci.

Dans certains modes de réalisation, le modèle chimique est un modèle cellulaire obtenu à partir d'un tissu nerveux, de préférence cérébral, d'une culture organotypique obtenue à partir d'un tissu cérébral, d'une culture de cellules nerveuses, par exemple de neurones de préférence dopaminergiques et/ou de cellules gliales, ou d'une lignée cellulaire, de préférence d'origine neuronale. Par exemple, le modèle chimique peut être obtenu à partir d'une lignée cellulaire choisie parmi le groupe constitué par les lignées SH-SY5Y, SK-N-MC et PC12.

Dans d'autres modes de réalisation, le modèle chimique est un animal non-humain, de préférence choisi dans le groupe constitué par un mammifère, un invertébré tel que C. *elegans* ou un poisson tel que le poisson zèbre.

L'invention concerne également un modèle chimique d'une synucléinopathie obtenu par la méthode ci-dessus décrite.

Ce modèle chimique peut être utilisé pour la mise en oeuvre d'un test de criblage de composés destinés au traitement ou à la prévention de ladite synucléinopathie, de préférence de la maladie de Parkinson ou encore pour évaluer l'efficacité d'un principe actif dans le traitement ou la prévention de ladite synucléinopathie, ledit modèle étant alors, de préférence, un modèle mammifère non-humain.

Un objet supplémentaire selon l'invention est un procédé pour évaluer, cribler ou sélectionner un composé destiné au traitement ou à la prévention d'une synucléinopathie, de préférence la maladie de Parkinson, ledit procédé comprenant:
a) la mise en contact d'un système cellulaire avec un composé tel que décrit précédemment, de préférence le pyridinium de furosémide,
b) la mise en contact dudit système cellulaire avec le composé à tester, et
c) la détection ou la quantification d'un marqueur caractéristique de la synucléinopathie en vue de déterminer l'efficacité du composé à tester,
l'étape b) pouvant être mise en oeuvre simultanément, avant ou après l'étape a) ou chevaucher l'étape a).

Ledit système cellulaire est choisi parmi une cellule, une culture cellulaire, un tissu et un animal non-humain et l'étape a) mise en oeuvre dans des conditions permettant l'obtention du modèle chimique selon l'invention en l'absence du composé à tester.

Dans certains modes de réalisation du procédé, le système cellulaire est choisi parmi le groupe constitué par un tissu nerveux, de préférence cérébral, une culture organotypique obtenue à partir d'un tissu cérébral, une culture de cellules nerveuses, par exemple de neurones de préférence dopaminergiques et/ou de cellules gliales, et une lignée cellulaire, de préférence d'origine neuronale telle que la lignée SH-SY5Y, et le marqueur est choisi parmi le groupe constitué par une accumulation intracellulaire d'une synucléine, de préférence d'a-synucléine, un marqueur de la mort cellulaire par apoptose, de préférence une augmentation d'une activité caspase, une diminution du taux de survie cellulaire, une diminution de l'activité de la chaîne respiratoire mitochondriale et leurs combinaisons. Dans d'autres modes de réalisation du procédé, le système cellulaire est un animal non-humain et le marqueur caractéristique de la synucléinopathie est choisi parmi le groupe constitué par un trouble moteur, en particulier un trouble de la mobilité ou une diminution de la vitesse de déplacement, une dégénérescence des neurones dopaminergiques, l'accumulation intracellulaire d'alpha-synucléine au niveau d'un neurone dopaminergique, une diminution de l'activité mitochondriale, en particulier du complexe I, et leurs combinaisons.

Un objet supplémentaire de l'invention est un kit pour la mise en oeuvre d'un procédé pour évaluer, cribler ou sélectionner un composé destiné au traitement ou à la prévention d'une synucléinopathie, tel que décrit ci-avant, ou pour la préparation d'un modèle chimique tel que décrit ci-avant comprenant:
- un composé tel que décrit précédemment, de préférence le pyridinium de furosémide ou l'un de ses sels acceptables sur la plan pharmaceutique, et
- un système cellulaire choisi parmi une cellule, une culture cellulaire, un tissu et un animal non-humain destiné à être mis en contact avec le composé pour obtenir ledit modèle chimique.

Dans le procédé ou kit selon l'invention, le système cellulaire peut être, par exemple, un nématode tel que C. *elegans,* un poisson zèbre, y compris une larve de ceux-ci. L'invention a également pour objet l'utilisation d'un composé tel que décrit précédemment, de préférence le pyridinium de furosémide ou l'un de ses sels acceptables sur le plan pharmaceutique, pour induire, chez un mammifère non-humain, un ou plusieurs symptômes associés à la maladie de Parkinson, de préférence choisi parmi le groupe constitué par une rigidité musculaire, un tremblement au repos, une instabilité posturale, une akinésie, un trouble du sommeil ou de l'éveil, une accumulation intracellulaire d'alpha-synucléine, de préférence sous la forme de corps de Lewy, et une dégénérescence des neurones dopaminergiques de la *substantia nigra.*

L'invention concerne l'utilisation d'un composé tel que décrit précédemment, de préférence le pyridinium de furosémide ou l'un de ses sels acceptables sur le plan pharmaceutique en tant que composé témoin, ou en tant que composé étalon, dans un test d'évaluation de la toxicité, de préférence la neurotoxicité, de composés, par exemple de polluants environnementaux.

### Figures

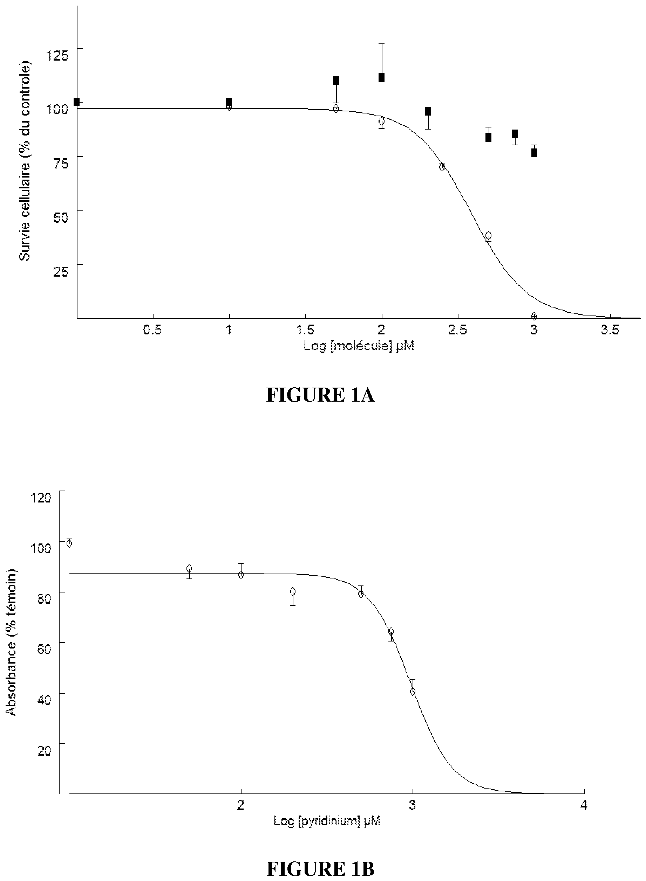

**La** **Figure 1A** présente les courbes d'effet-dose réponse :
   - du taux de survie cellulaire de la lignée SH-SY5Y après une incubation de 24h en présence de pyridinium de furosémide (carrés noirs).
   - du taux de survie cellulaire de la lignée SH-SY5Y après une incubation de 6h en présence de MPP+ plus 18h sans MPP+ (ronds blancs).

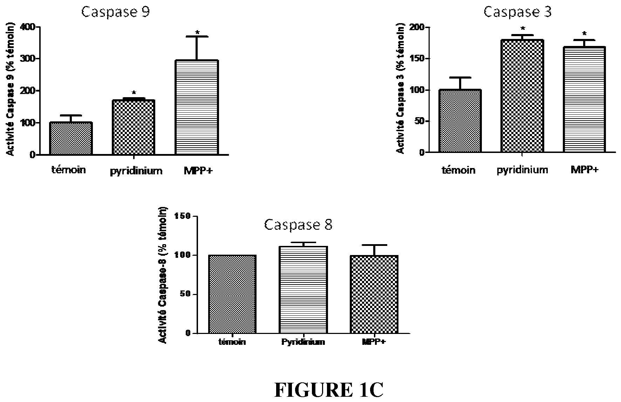

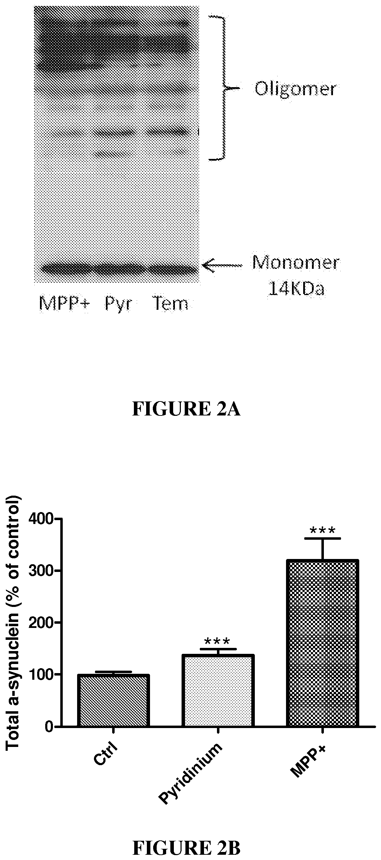

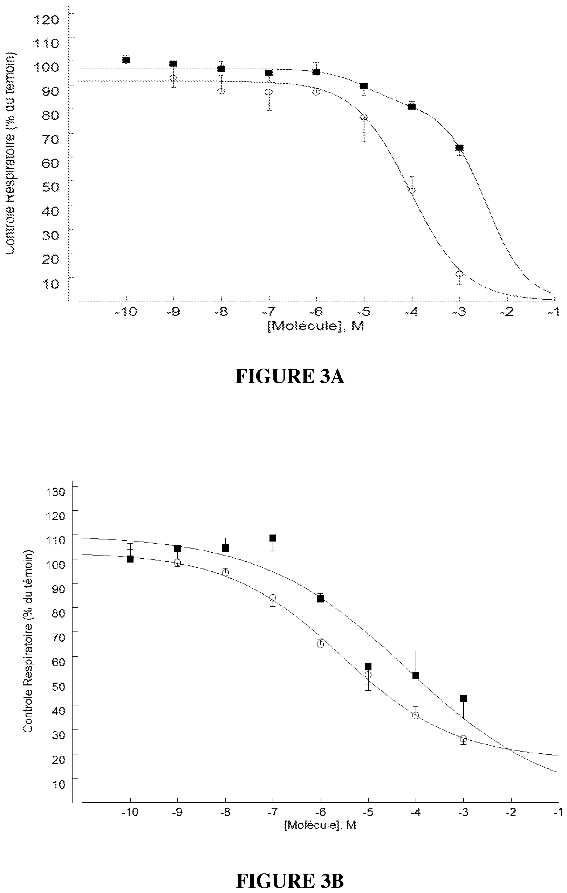

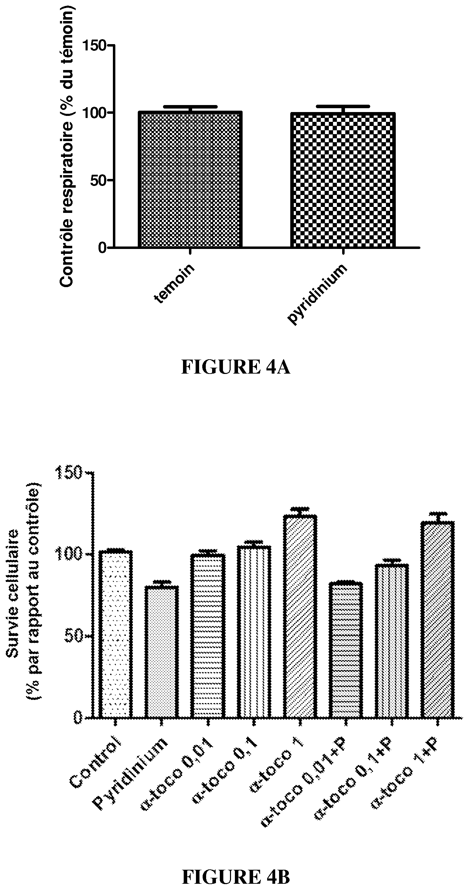

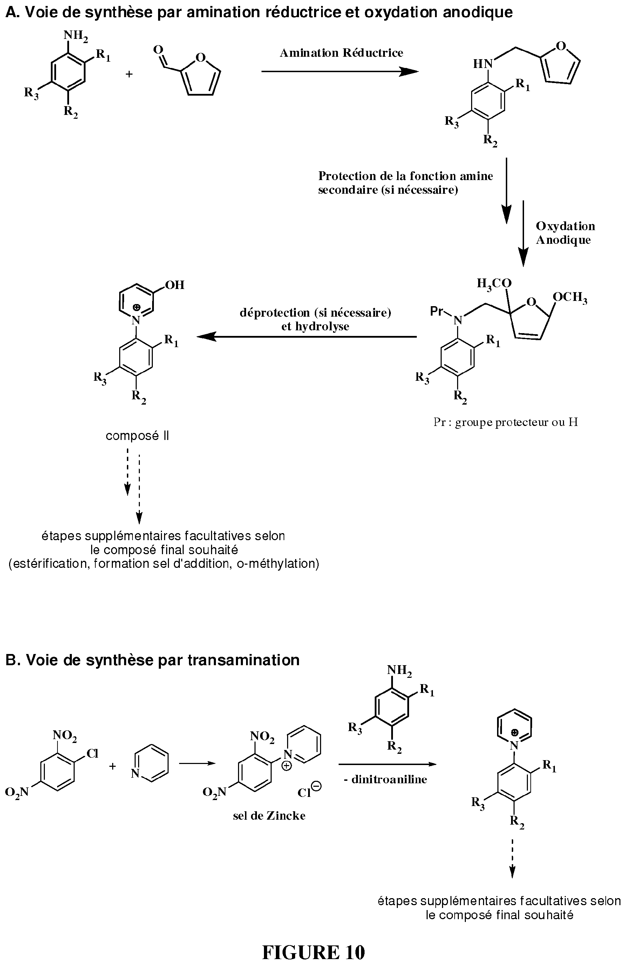

   Le taux de survie cellulaire est déterminé par le test de viabilité au MTT comme détaillé dans les exemples. Les résultats obtenus sont normalisés par rapport à l'absorbance obtenue pour des cellules SH-SY5Y non exposées au pyridinium de furosémide ou au MPP+.
   Ordonnées : % d'absorbance à 550 nm, Abscisses : logarithme de la concentration de pyridinium de furosémide ou du MPP+ exprimée en µM.
**La** **Figure 1B** présente la courbe d'effet-dose réponse de la concentration de pyridinium de furosémide sur le taux de survie cellulaire de la lignée SH-SY5Y après une incubation de 96h. Le taux de survie cellulaire est déterminé par le test de viabilité au MTT comme détaillé dans les exemples. Les résultats obtenus sont normalisés par rapport à l'absorbance obtenue pour des cellules SH-SY5Y non exposées au pyridinium de furosémide.
   Ordonnées : % d'absorbance à 550 nm, Abscisses : logarithme de la concentration de pyridinium de furosémide exprimée en µM.
**La** **Figure 1C** présente 3 diagrammes présentant les activités caspase détectées pour l'expérience témoin (témoin), après incubation avec le pyridinium de furosémide (pyridinium) pendant 96 h ou après incubation avec le MPP+ (MPP+) pendant 24 h. Ordonnées : Activité caspase exprimée en pourcentage par rapport à l'expérience témoin. Diagramme haut, gauche : caspase 9. Diagramme haut, droite : caspase 3. Diagramme bas : caspase 8.
**La** **Figure 2A** montre le gel obtenu par western blot après révélation avec un anticorps primaire anti-synucléine et un anticorps secondaire couplé à la HRP pour des cellules SH-SY5Y incubées en présence de MPP+ pendant 24 h, de pyridinium de furosémide pendant 96 h ou sans composé. TEM : témoin, MPP+ : MPP+, pyridinium : pyridinium de furosémide, oligomer : oligomère d'alpha-synucléine, monomer : monomère d'alpha-synucléine.
**La** **Figure 2B** est un diagramme montrant la quantité relative d'α-synucléine accumulée par les cellules après incubation avec le pyridinium de furosémide (pyridinium) pendant 96 h ou après incubation au MPP+ pendant 24 h en comparaison avec la quantité d'α-synucléine présente dans les cellules témoins (Ctrl). Ordonnées : pourcentage d'α-synucléine par rapport à la quantité détectée dans les cellules témoins - quantification des gels western blot.
**La** **Figure 3A** montre l'effet dose-réponse du MPP+ (ronds blancs) ou du pyridinium de furosémide (carrés noirs) sur le contrôle respiratoire des mitochondries isolées à partir du cortex cérébral de rat. Ordonnées : pourcentage de contrôle respiratoire (%) exprimé par rapport à l'expérience témoin (mitochondries non exposées aux molécules). Abcisses: logarithme de la concentration en mole.
**La** **Figure 3B** montre l'effet dose-réponse du MPP+ (ronds blancs) ou du pyridinium de furosémide (carrés noirs) sur le contrôle respiratoire des mitochondries isolées à partir du stratium de rat. Ordonnées : pourcentage de contrôle respiratoire (%) exprimé par rapport celui de l'expérience témoin (mitochondries non exposées aux molécules). Abcisses: logarithme de la concentration en mole.
**La** **Figure 4A** est un diagramme montrant le pourcentage de contrôle respiratoire des mitochondries témoin et celles exposées au pyridinium de furosémide après induction du complexe II par le succinate et inhibition du complexe I par la roténone.
**La** **Figure 4B** est un diagramme montrant le pourcentage de survie cellulaire, par rapport aux cellules témoins (control), de cellules SH-SY5Y ayant subi différents traitements. De gauche à droite : contrôle ; incubation avec 0,5mM de pyridinium de furosémide (pyridinium) ; pré-incubation avec 0,01 µg/ml d'α-tocophérol (α-toco) ; pré-incubation avec 0,1 µg/ml d'α-tocophérol ; pré-incubation avec 1 µg/ml d'α-tocophérol ; pré-incubation avec 0,01 µg/ml d'α-tocophérol + incubation 0,5 mM de pyridinium de furosémide ; pré-incubation avec 0,1 µg/ml d'α-tocophérol + incubation 0,5 mM de pyridinium de furosémide ; et pré-incubation avec 1 µg/ml d'α-tocophérol + incubation 0,5 mM de pyridinium de furosémide. (Durée de la pré-incubation avec l'α-tocophérol : 24 h, durée de l'incubation avec le pyridinium de furosémide : 96 h).

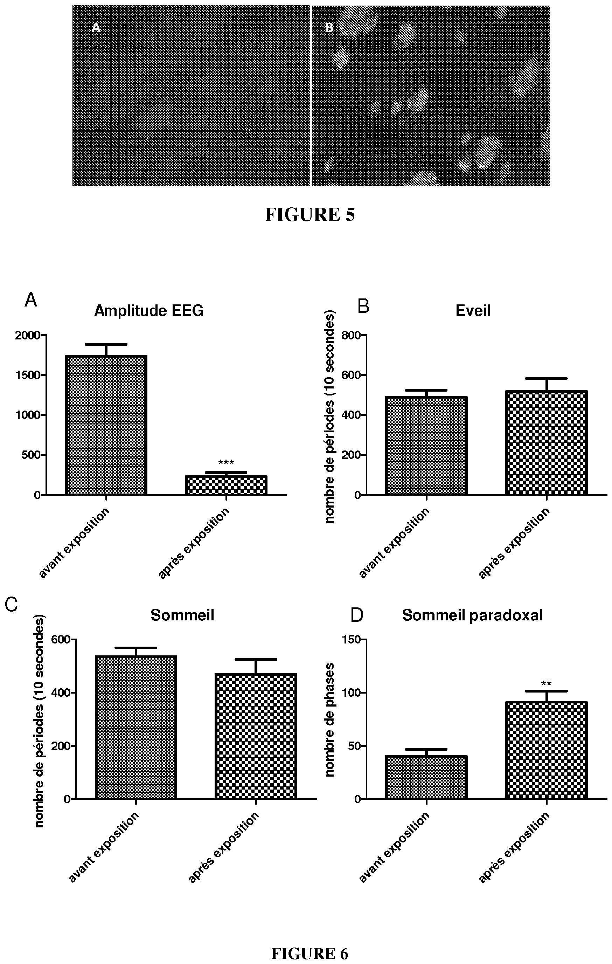

Les **Figures 5 et 6** illustrent les effets de l'administration de pyridinium de furosémide chez le rat, à raison d'une dose orale journalière de 20 mg/kg pendant 7 jours. Les rats sont sacrifiés 5 semaines après la dernière administration de pyridinium de furosémide (voir Exemples, Partie C, point 1).
**La** **Figure 5** présente l'immunomarquage de coupes coronales du stratium de rats non-exposés au pyridinium de furosémide (Figure 5A) et exposés au pyridinium de furosémide (Figure 5B) avec un anticorps dirigé contre l'alpha-synucléine. Le marquage de l'alpha-synucléine pour les coupes provenant de rats exposés au pyridinium de furosémide (Figure 5B) est nettement plus important que celui observé pour les coupes provenant de rats non exposés et révèle la présence de vésicules. L'exposition au furosémide de pyridinium induit donc une accumulation d'alpha-synucléine sous la forme de vésicules au niveau du stratium cérébral.
**La** **Figure 6** montre les résultats issus de l'enregistrement de l'électroencéphalogramme (EEG) chez un rat avant l'administration du pyridinium de furosémide (histogrammes « avant exposition ») et 5 semaines après la fin du traitement au pyridinium de furosémide (histogrammes « après exposition »). **Figure 6A** **:** amplitude de l'EEG, **Figure 6B** : nombre de périodes d'éveil (supérieures à 10 s), **Figure 6C** : nombre de périodes de sommeil (supérieures à 10 s) et **Figure 6D** : nombre de phases de sommeil paradoxal. Après exposition au pyridinium de furosémide, on observe une nette diminution de l'amplitude de l'EEG et une augmentation du nombre de phases de sommeil paradoxal.

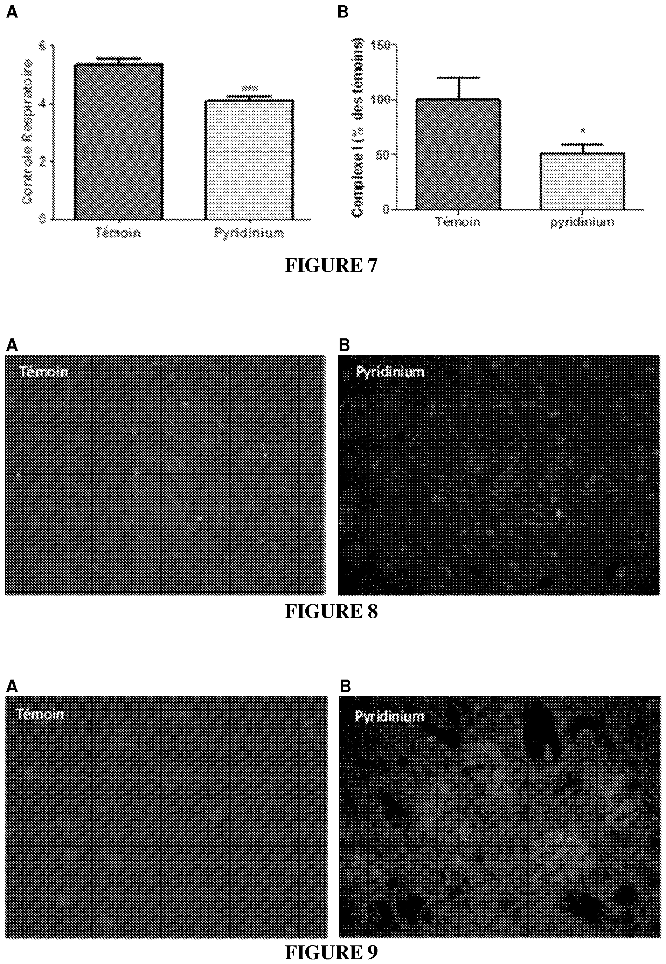

Les **Figures 7 à 9** montrent les effets de l'administration d'une dose journalière de 20 mg/kg, par voie orale, pendant 7 jours, chez la souris. Les souris ont été sacrifiées 30 jours après la fin de l'exposition au pyridinium de furosémide (voir partie Exemples, Partie C, point 2).
**La** **Figure 7** montre le niveau d'activité de la chaine respiratoire (Figure 7A) et du complexe I (Figure 7B) pour des mitochondries isolées à partir du stratium de souris ayant été exposées au pyridium de furosémide par rapport aux niveaux d'activité mesurés pour des mitochondries provenant du stratium des souris témoins. Les souris qui ont été exposées au pyridinium de furosémide présentent une diminution nette du contrôle respiratoire et du complexe I mitochondrial. L'exposition au pyridinium de furosémide induit un dysfonctionnement de la chaîne respiratoire mitochondriale au niveau du stratium, qui est encore détectable 30 jours après la fin de l'exposition.
**La** **Figure 8** montre l'immunomarquage, par un anticorps dirigé contre la tyrosine hydroxylase, d'une coupe coronale provenant du stratium d'une souris témoin (Figure 8A - témoin) et d'une coupe coronale provenant du stratium d'une souris exposée au pyridinium de furosémide (Figure 8B - pyridinium). Le marquage de la tyrosine hydroxylase pour la coupe coronale provenant de la souris exposée au pyridinium de furosémide est beaucoup plus faible que le marquage détecté sur la coupe coronale de la souris témoin.
**La** **Figure 9** montre l'immunomarquage, par un anticorps dirigé contre l'alpha-synucléine phosphorylée en Ser129, d'une coupe coronale provenant du stratium d'une souris témoin (Figure 9A - témoin) et d'une coupe coronale provenant du stratium d'une souris exposée au pyridinium de furosémide (Figure 9B - pyridinium). Le marquage de l'alpha-synucléine phosphorylée est beaucoup plus important pour la coupe coronale provenant de la souris exposée au pyridinium de furosémide que pour la coupe coronale de la souris témoin.
**La** **Figure 10A** propose un schéma réactionnel pour la synthèse des composés de formule (I) dans laquelle R₄ est -OH ou -OMe. A l'issue de l'étape d'amination réductrice, il est recommandé de protéger la fonction amine secondaire avant d'entreprendre l'étape d'oxydation anodique. Une telle étape de protection n'est pas nécessaire pour la synthèse du pyridinium de furosémide. Pr représente un groupe protecteur de la fonction amine ou un hydrogène.
La Figure 10B propose un schéma réactionnel pour la synthèse de composés de formule (I) dans laquelle R₄ est H.

Les groupements R₁, R₂ et R₃ sont tels que définis dans la description.

### Description détaillée de l'invention

### Utilisation du pyridinium de furosémide, méthode pour la préparation d'un modèle chimique d'une maladie neurodégénérative et kit

La présente invention concerne un nouveau modèle chimique d'une maladie neurodégénérative, de préférence d'une synucléinopathie, basé sur l'utilisation du pyridinium de furosémide ou de l'un de ses sels, prodrogues, métabolites ou dérivés, en tant qu'agent chimique.

Le furosémide est un agent diurétique de l'anse commercialisé sous le nom de Lasix® pour le traitement de l'hypertension et de l'oedème associé à l'insuffisance cardiaque globale. Il est excrété par l'organisme majoritairement sous une forme inchangée.

Son oxydation *in vitro* par les microsomes hépatiques en un dérivé pyridinium a été décrite par Chen, et al., (ChemResToxicol. 2007,20:1741-4). Ce dérivé pyridinium (appelé ci-après pyridinium de furosémide) est considéré comme un métabolite potentiel du furosémide pouvant exercer une activité diurétique. A la connaissance du Demandeur, aucune autre activité biologique du pyridinium de furosémide n'a été documentée dans l'état de la technique, à ce jour.

De manière surprenante, le Demandeur a montré que le pyridinium de furosémide présentait une neurotoxicité *in vitro,* plus exactement, qu'il réduisait la viabilité cellulaire de la lignée humaine neuronale SH-SY5Y et ceci de manière dose-dépendante. A cet égard, le Demandeur précise que la lignée SH-SY5Y est un modèle cellulaire de neurone dopaminergique largement utilisé et reconnu par la communauté scientifique.

Le Demandeur a également montré que l'incubation des cellules neuronales SH-SY5Y avec le pyridinium de furosémide induisait une augmentation nette des activités caspase 9 et caspase 3, suggérant une mort cellulaire impliquant la voie intrinsèque mitochondriale de l'apoptose. Par ailleurs, le Demandeur a observé que l'incubation avec le furosémide de pyridinium induit, chez les cellules, une accumulation d'α-synucléine avec apparition d'entités oligomériques et dimériques. Le Demandeur a également montré que le pyridinium de furosémide affecte le fonctionnement de la chaîne respiratoire mitochondriale en inhibant sélectivement le complexe I.

Ces résultats montrent que le pyridinium de furosémide est capable d'induire au niveau cellulaire des phénomènes biologiques - inhibition du complexe I mitochondrial, mort cellulaire par apoptose, et accumulation intra-cellulaire d'α-synucléine - similaires à ceux observés dans le modèle chimique MPTP/MPP+ qui est le modèle de référence utilisé pour l'étude de la maladie de Parkinson. En d'autres termes, le pyridinium de furosémide induit au niveau cellulaire des phénomènes analogues à ceux observés avec le MPP+. Néanmoins, le pyridinium de furosémide présente une cinétique de toxicité significativement plus lente que celle du MPP+. En effet, une incubation des cellules SH-SY5Y de 96h avec le pyridinium de furosémide est nécessaire pour obtenir un taux de mortalité cellulaire analogue à celui observé pour une incubation de 6 h avec le MPP+. Une telle incubation avec le pyridinium de furosémide induit une augmentation du taux intracellulaire d'α-synucléine beaucoup plus faible que celui observé par incubation avec le MPP+, mais ce taux reste significativement plus élevé que celui observé pour l'expérience témoin. Par ailleurs, le furosémide de pyridinium présente une plus grande spécificité vis-à-vis du complexe I mitochondrial du stratium que le MPP+. Ces résultats *in vitro* ont été confirmés *in vivo* par les essais réalisés par le Demandeur chez le rat et la souris. L'administration d'une dose journalière de 20 mg/kg de pyridinium de furosémide sur sept jours n'a pas été létale, mais a entrainé une dysfonction de la chaîne respiratoire mitochondriale au niveau du stratium et l'apparition de marqueurs histologiques propres à la maladie de Parkinson, à savoir une accumulation intracellulaire spécifique d'alpha-synucléine sous la forme de vésicules, au niveau du stratium. Chez la souris, le Demandeur a également mis en évidence une augmentation très nette de l'expression de l'alpha-synucléine phosphorylée en Ser129 qui est la forme majoritaire présente dans les corps de Lewy chez l'homme. A la connaissance du Demandeur, un tel résultat n'a pas été observé, à ce jour, pour les autres modèles chimiques de la maladie de Parkinson.

Le Demandeur a également observé, chez le rat, l'apparition de troubles du sommeil - à savoir une augmentation du nombre de phases de sommeil paradoxal - qui peuvent être observés chez des patients souffrant de synucléinopathies.

De par sa toxicité plus faible et sa plus grande spécificité vis-à-vis du stratium, le pyridinium de furosémide permet d'obtenir des modèles chimiques animaux mimant de manière plus fidèle les synucléinopathies, en particulier en ce qui concerne la progressivité, les symptômes et/ou les marqueurs histologiques de ces maladies. Le pyridinium de furosémide et ses dérivés trouvent donc des applications dans l'étude du développement - en particulier des stades précoces - des maladies neurodégénératives, en particulier des synucléinopathies, et dans l'identification de nouveaux traitements permettant de ralentir, de bloquer voire de guérir ces pathologies.

Ainsi, un premier objet de la présente invention concerne l'utilisation d'un agent chimique choisi parmi le groupe constitué par le pyridinium de furosémide, un métabolite, un dérivé, une prodrogue de celui-ci, leurs sels et leurs combinaisons, dans la préparation d'un modèle chimique d'une maladie neurodégénérative.

On entend par « pyridinium de furosémide » le composé chimique de formule suivante:

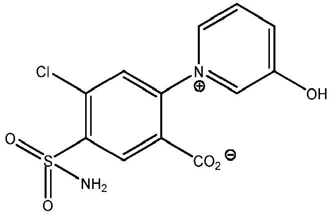

Le nom chimique du pyridinium de furosémide est 4-chloro-2-(3-hydroxypyridinium-1-yl)-5-sulfamoylbenzoate. Il est à noter que le pyridinium de furosémide se présente sous forme d'un zwitterion mais peut également se présenter sous forme de sels d'addition.

Le pyridinium de furosémide peut être obtenu à partir du furosémide par oxydation anodique comme décrit par les inventeurs dans Laurencé et al., Tetrahedron, 20011, 67, 9518-9521.

Au sens de l'invention, une «prodrogue du pyridinium de furosémide» désigne un composé dont la métabolisation *in vivo,* ou *in vitro* - c'est-à-dire par une cellule ou par une machinerie cellulaire - conduit à la formation de pyridinium de furosémide. On entend par « machinerie cellulaire », toute protéine, en particulier toute enzyme, organite ou complexe supramoléculaire (tels que les microsomes) obtenus à partir d'une cellule.

A titre d'exemple, le furosémide, qui peut être métabolisé *in vitro* par les microsomes hépatiques, est une prodrogue du pyridinium. De préférence, les prodrogues du pyridinium de furosémide sont choisies parmi les esters de celui-ci.

On entend par « métabolite du pyridinium de furosémide» tout composé issu de la métabolisation *in vitro* ou *in vivo* du pyridinium de furosémide, ledit métabolite présentant, de préférence des activités biologiques similaires à celles du pyridinium de furosémide.

Au sens de l'invention, un « dérivé du pyridinium du furosémide » désigne un composé qui ne diffère structurellement du pyridinium de furosémide que par une modification structurale mineure. De préférence, ledit métabolite ou dérivé est capable, *in vitro,* de diminuer le taux de survie cellulaire, d'augmenter les activités caspase, en particulier la caspase 3 et/ou 9, d'inhiber le complexe I mitochondriale et/ou d'induire une accumulation intracellulaire d'alpha-synucléine chez la lignée cellulaire SH-SY5Y. Le métabolite ou dérivé présente, de préférence, ces quatre caractéristiques.

Dans certains modes de réalisation de l'invention, l'agent chimique est un composé de formule (I): 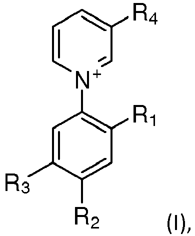 dans laquelle,
- R₁ représente un atome d'hydrogène, un atome d'halogène, un alkyle en C₁-C₄, - OH, -CN, -C(=O)NH₂, -CF₃, -C(=O)O⁻, ou -C(=O)O-R₅ avec R₅ représente -H ou un alkyle en C₁-C₄,
- R₂ est -SO₂NH₂, un atome d'hydrogène, un atome d'halogène, un alkyle en C₁-C₄,-OH, -CN, -C(=O)NH₂, -CF₃, ou -C(=O)OR₆ avec R₆ représente H ou un alkyle en C₁-C₄
- R₃ est H, un atome d'halogène, un alkyle en C₁-C₄,-OH, -CN, -C(=O)NH₂, -CF₃, ou -C(=O)OR₇ avec R₇ représente H ou un alkyle en C₁-C₄. et
- -R₄ représente un atome d'hydrogène, -OH ou -OCH₃,
ou un sel pharmaceutiquement acceptable de celui-ci.

Au sens de l'invention, un atome d'halogène englobe Br, Cl, I ou F, de préférence Br et Cl. Un groupe alkyle en C₁-C₄ englobe les groupes méthyle, éthyle, propyle et butyle, de préférence un groupe méthyle.

Il va de soi que l'agent chimique comprend le ou les contre-anions ou contre-cations nécessaires en fonction de l'état d'ionisation des goupes R₁, R₂, R₃ et R₄.

Dans certains modes de réalisation de l'invention, l'agent chimique est un composé de formule (I) dans laquelle :
- R₁ est -H, un atome d'halogène, -CN, -C(=O)NH₂, -CF₃, -C(=O)O⁻, ou -C(=O)OR₅ avec R₅ représente -H ou un alkyle en C₁-C₄,
- R₂ est -C(=O)NH₂, -SO₂NH₂, un atome d'halogène, -H, -CN, ou -CF₃,
- R₃ est -H, un atome d'halogène, -CN, -CONH₂, -CF₃, -C(=O)O⁻, ou -C(=O)OR₇ avec R₇ représente -H ou un alkyle en C₁-C₄, et
- R₄ est -OH, -OMe, ou -H.

Dans d'autres modes de réalisation de l'invention, l'agent chimique est un composé de formule (I) dans laquelle :
- R₁ est -H, un atome d'halogène, -CN, -C(=O)NH₂, -CF₃, -C(=O)O⁻, ou -C(=O)OR₅ avec R₅ représente -H ou un alkyle en C₁-C₄,
- R₂ est -C(=O)NH₂, -SO₂NH₂, un atome d'halogène, -H, -CN, ou -CF₃,
- R₃ est -H, un atome d'halogène, -CN, -CONH₂, -CF₃, et
- R₄ est -OH, -OMe, ou -H.

Dans un autre mode de réalisation, l'agent chimique est un composé de formule (I) dans laquelle :
- R₁ représente un atome d'hydrogène, -C(=O)O⁻, ou -C(=O)O-R₅ où R₅ est H ou un groupe alkyle en C₁-C_{4,},
- R₂ est -SO₂NH₂, -CONH₂, un atome d'hydrogène, ou un atome d'halogène,
- R₃ est -H ou un atome d'halogène, et
- R₄ est -OH, -OMe ou -H
ou un sel acceptable sur le plan pharmaceutique de celui-ci.

Dans certains modes de réalisation, l'agent chimique est un composé de formule (I) dans lequel R₄ est -OH. En particulier, l'agent chimique peut être un composé de formule (la): 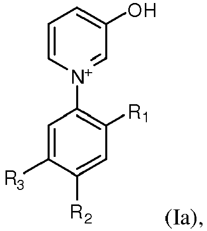 Dans laquelle,
- R₁ représente un atome d'hydrogène, -C(=O)O⁻, ou -C(=O)O-R₅ où R₅ est H ou un groupe alkyle en C_{1-4,},
- R₂ est -SO₂NH₂, un atome d'hydrogène, ou un atome d'halogène, et
- R₃ est H ou un atome d'halogène,
ou un sel acceptable sur le plan pharmaceutique de celui-ci.

Il va de soi que lorsque R₁ est H ou -C(=O)OR₅, le composé de formule (la) est associé à un contre-anion Q⁻ acceptable sur le plan pharmaceutique. A titre d'exemple, Q⁻ peut être choisi parmi Cl⁻, Br⁻, CH₃COO⁻ ou SO₄²⁻.

Lorsque R₁ est COO⁻, le composé est sous forme zwittérion et Q⁻ est absent

Dans un mode de réalisation particulier, l'agent chimique est un composé de formule (la) dans laquelle :
- R₁ représente un atome d'hydrogène ou -C(=O)O⁻,
- R₂ est -SO₂NH₂, un atome d'hydrogène, -Br ou -Cl, et
- R₃ est H, -Br ou -Cl,
ou un sel acceptable sur le plan pharmaceutique de celui-ci.

A titre d'exemple, l'agent chimique peut être choisi parmi les composés suivants: 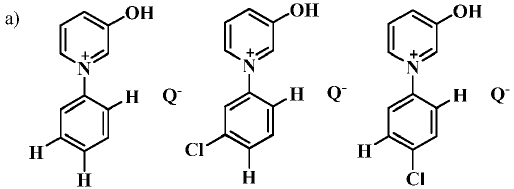 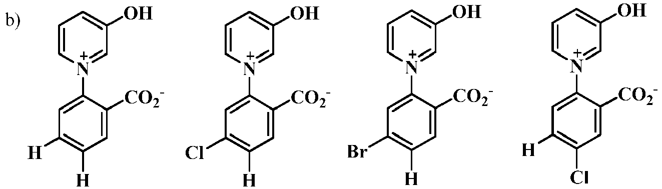 ou les sels d'additions des composés b).

Les composés de formule (I) pour lesquels R₄ est -OH ou -OMe peuvent être obtenus à partir de dérivés d'aniline ou d'acide anthraniliques disponibles dans le commerce ou facilement accessibles par synthèse chimique. Typiquement, le dérivé d'aniline ou d'acide anthranilique subit une première étape d'amination réductrice en présence de furfural puis une étape d'oxydation par voie électrochimique et/ou chimique conduisant en un dérivé diacétal. Le dérivé diacétal est converti en dérivé pyridinium (composé (II), Figure 10) après réarrangement intramoléculaire par hydrolyse acide. Selon le composé final souhaité, le dérivé de pyridinium peut éventuellement subir une étape d'estérification ou de méthylation. A l'issue de l'étape d'amination réductrice, il est généralement nécessaire de protéger l'amine secondaire formée avant la mise en oeuvre de l'étape d'oxydation. Le groupe protecteur est généralement clivé avant la mise en oeuvre de l'étape de cyclisation. Lorsque le groupe protecteur introduit est clivable en milieu acide, les étapes de déprotection et de cyclisation peuvent être réalisées simultanément. A cette fin, un groupe protecteur adapté est le groupe BOC (tert-butoxycarbonyle). Comme illustré dans les exemples (voir partie A), il n'est pas nécessaire de protéger la fonction amine secondaire pour la synthèse du pyridinium de furosémide.

Les composés de formule (I) pour lesquels R₄ est -H peuvent être obtenus par réaction de transamination. Cette voie de synthèse comprend la préparation d'un sel de Zincke par réaction de la pyridine et du 1-chloro-2,4-dinitrobenzène, et la réaction dudit sel de Zincke avec un dérivé d'aniline de manière à obtenir le pyridinium souhaité.

De préférence, on utilise, pour la mise en oeuvre de l'invention selon ses différents aspects (voir ci-après), le pyridinium de furosémide ou l'un de ses sels acceptables sur le plan pharmaceutique.

Dans ce qui suit, le terme générique « agent chimique » désigne un composé choisi parmi le groupe constitué par le pyridinium de furosémide, un métabolite, un dérivé, une prodrogue de celui-ci, leurs sels et leurs combinaisons, de préférence un composé de formule (I) ou (la) tel que décrit ci-avant et de manière encore plus préférée, le pyridinium de furosémide ou l'un de ses sels acceptables sur le plan pharmaceutique.

On entend par « maladie neurodégénérative », toute maladie se caractérisant par une dégénérescence d'une ou de plusieurs populations cellulaires du système nerveux. Dans le contexte de l'invention, la maladie neurodégénérative se caractérise, de préférence, par une dégénérescence d'une population de neurones cérébraux. A titre d'exemple de neurones cérébraux d'intérêt, on peut citer les neurones dopaminergiques de la *substantia nigra* ou du cortex cérébral. Dans un mode de réalisation préféré de l'invention, la maladie neurodégénérative est une synucléinopathie.

Au sens de l'invention, on entend par « synucléinopathie », toute maladie neurodégénérative se caractérisant par une accumulation intracellulaire anormale d'une synucléine. De préférence, la synucléine est l'alpha-synucléine, ladite alpha-synucléine s'accumulant sous forme d'agrégats, par exemple, sous la forme de neurites de Lewy et/ou au sein d'inclusions cytoplasmiques telles que les corps de Lewy. Les synucléinopathies selon l'invention, englobent, sans y être limitées, la maladie de Parkinson, l'atrophie multi-systématisée (multiple system atrophy en anglais), la démence à corps de Lewy, la maladie d'Hallervorden-Spatz, le syndrome de Sanfilippo (Mucopolysaccharidose de type IIIA), la maladie de Niemann-Pick de type C. Dans un mode de réalisation préféré, la maladie neurodégénérative est la maladie de Parkinson, en particulier une forme sporadique de la maladie de Parkinson.

On entend par « modèle chimique d'une maladie» ou par « modèle toxique d'une maladie », un système cellulaire qui, après mise en contact avec un agent chimique spécifique, présente un ou plusieurs mécanismes biologiques, un ou plusieurs marqueurs biologiques et/ou un ou plusieurs symptômes associés à ladite maladie. On désigne par « mise en contact » ou « par exposition » l'incubation du système cellulaire avec l'agent chimique ou encore l'administration dudit agent chimique au système cellulaire.

Dans le cadre spécifique de la présente invention, l'agent chimique capable d'induire chez le système cellulaire les phénomènes souhaités est tel que décrit précédemment, de préférence un composé de formule (I).

Au sens de l'invention, un « système cellulaire » désigne une entité choisie parmi le groupe constitué par une cellule, une culture cellulaire, un tissu ou un animal non-humain. Il est à noter qu'au sens de l'invention, un système cellulaire, et donc un modèle chimique selon l'invention, ne comprend pas les êtres humains, les embryons humains, et les cellules souches embryonnaires humaines.

Dans certains modes de réalisation, le modèle chimique selon l'invention peut être un modèle chimique cellulaire, c'est-à-dire une cellule eucaryote, une culture cellulaire ou encore un tissu. Il est entendu que le terme « tissu» se réfère à un ensemble de cellules organisées entre elles, qui a été isolé à partir d'un être vivant.

De préférence, le modèle chimique cellulaire est obtenu à partir d'une culture cellulaire, d'une cellule d'origine neuronale,d'une lignée cellulaire de préférence d'origine neuronale, ou d'un tissu nerveux, de préférence cérébral.

Au sens de l'invention, « une culture cellulaire » comprend, sans y être limitée, une culture cellulaire d'une lignée cellulaire, une culture primaire, une culture mixte (ou co-culture) comprenant plusieurs types cellulaires, et une culture organotypique.

Le choix de la culture cellulaire ou du tissu dépendra de la maladie neurodégénérative, en particulier de la synucléinopathie visée.

Pour obtenir un modèle cellulaire chimique de la maladie de Parkinson, on peut, par exemple, utiliser une culture primaire de neurones dopaminergiques, une culture organotypique dérivé d'un tissu cérébral, une culture mixte comprenant plusieurs types cellulaires d'origine neuronale, par exemple des neurones et/ou des cellules gliales telles que les astrocytes et les oligodendrocytes, ou une lignée cellulaire, de préférence d'origine neuronale, présentant une ou plusieurs caractéristiques biochimiques des neurones dopaminergiques telles que l'expression d'une tyrosine hydroxylase, d'une dopamine-beta-hydroxylase et/ou du transporteur de la dopamine. Il peut s'agir d'une lignée cellulaire de neuroblastome. De telles lignées cellulaires, comprennent sans y être limitées, la lignée de neuroblastome humaine SH-SY5Y (N° d'enregistrement ATCC : CRL-2266), la lignée humaine de neuroblastome SK-N-MC (N°ATCC : HTB-10) ou la lignée cellulaire murine PC12 (N° d'enregistrement ATCC : CRL-1721).

Pour un exemple de mise en oeuvre de culture primaire de neurones dopaminergiques, on pourra se référer à l'article de Schmidt, F., et al. (PLoSone, 2009 4(7): e6215)

Dans d'autres modes de réalisation, le modèle chimique selon l'invention est un modèle chimique animal obtenu à partir d'un animal non-humain. Il peut s'agir d'un animal fréquemment utilisé en développement pharmaceutique ou en étude pré-clinique. L'animal peut être un mammifère, par exemple choisi parmi le groupe constitué par un primate tel que le babouin, un macaque (par exemple macaque rhesus ou macaque crabier), un singe vert africain, ou un chimpanzé, un rongeur tel qu'une souris ou un rat, et un canidé tel qu'un chien.

A titre alternatif, l'animal peut être un invertébré comme une drosophile ou un nématode, par exemple *Caenorhabditis elegans,* ou encore un poisson, tel que le poisson zèbre (*Danio rerio*)*.* Selon le modèle chimique recherché, on pourra utiliser une larve, un individu juvénile ou un individu adulte de ces animaux.

Le système cellulaire peut être une cellule ou un organisme génétiquement modifié. Il peut s'agir d'un système cellulaire dans lequel un gène hétérologue a été introduit afin de faciliter l'utilisation du modèle chimique final. A titre d'exemple, le système cellulaire peut être une souche de *C*. *elegans* ou de poisson zèbre exprimant un marqueur fluorescent tel que la GFP (Green Fluorescent Protein) au niveau des neurones dopaminergiques grâce au contrôle d'un promoteur tissu-spécifique. Il peut s'agir également d'une souche transgénique comportant une modification génétique la rendant plus sensible à l'agent chimique. Pour des exemples d'animaux transgéniques adaptés à la mise en oeuvre de l'invention, on pourra se référer à Blandini *et al.* (*supra*)*,* à Chen et al. (Developmental Dynamics, 2009, 238, 746-754) ou encore à Rubinstein (Expert Opinion Drug Metab, 2006, 2, 231-240).

Dans un second aspect, la présente invention a pour objet une méthode de préparation d'un modèle chimique d'une maladie neurodégénérative, de préférence un modèle chimique d'une synucléinopathie, et de manière encore plus préférée, un modèle chimique de la maladie de Parkinson.

La méthode de préparation selon l'invention est caractérisée en ce qu'elle comprend une étape de mise en contact d'un système cellulaire tel que défini précédemment avec l'agent chimique, de préférence choisi parmi le groupe constitué par les composés de formule (I), leurs sels et les combinaisons de ceux-ci.

Dans le cadre de la présente invention, la « mise en contact » peut désigner l'incubation du système cellulaire avec l'agent chimique d'intérêt, lorsque ledit système cellulaire est par exemple, une cellule, une culture cellulaire, un tissu ou un animal de petite taille tel qu'un nématode ou un poisson-zèbre. Dans ce mode de réalisation, le système cellulaire est mis en contact avec une solution contenant l'agent chimique pendant une durée pouvant aller de quelques minutes à plusieurs heures, voire plusieurs jours. La durée d'incubation peut dépendre de la concentration de l'agent chimique dans la solution. Typiquement, la solution d'incubation est un milieu de culture dans lequel l'agent chimique a été rajouté à la concentration désirée. La concentration de l'agent chimique est généralement comprise dans une gamme allant de 1.10⁻¹² à 1 mol.l⁻¹, de préférence de 1.10⁻¹⁰ à 1.10⁻¹ mol.l⁻¹. Une gamme de 1.10⁻¹⁰ à 1.10⁻² mol.l⁻¹ englobe une gamme allant de 1.10⁻¹⁰ à 1.10⁻⁹, 1.10⁻⁹ à 1.10⁻⁸,1.10⁻⁸ à 1.10⁻⁷, de 1.10⁻⁷ à 1.10⁻⁶, de 1.10⁻⁶ à 1.10⁻³, de 1.10⁻³ à 1.10⁻² mol-l⁻¹.

A titre alternatif, lorsque ledit système cellulaire est un animal de plus grande taille, tel qu'un rongeur, un primate ou un canidé, la « mise en contact » correspond à une étape d'administration de l'agent chimique audit système cellulaire.

L'agent chimique peut être administré par voie systémique ou par voie locale. Les voies d'administration adaptées comprennent, sans y être limitées, les voies orale, intraveineuse, intra-artérielle, intranasale, intradermique, sous-cutanée, intramusculaire, intrapéritonéale, intraoculaire et intracrânienne. Les voies d'administration préférées sont les voies orale, intraveineuse, sous-cutanée, intrapéritonéale, intranasale, et intracrânienne.

La dose à administrer et le schéma d'administration dépend de l'animal non-humain utilisé, en particulier, de son poids, de la voie d'administration choisie, et de sa sensibilité à l'agent chimique et de l'effet recherché. L'homme du métier saura, par des tests de routine, par exemple à travers l'analyse des troubles moteurs développés par l'animal ou à travers des études histologiques post-mortem, déterminer la dose et le schéma d'administration adéquate. Typiquement, la dose journalière à administrer peut varier de 200 ng/kg à 100 mg/kg, de préférence de 0,01 mg/kg à 30 mg/kg. Le schéma d'administration peut comprendre l'administration d'une dose unique ou de plusieurs doses sur plusieurs heures, voire plusieurs jours ou plusieurs semaines.

L'agent chimique peut être formulé à l'aide d'un véhicule acceptable sur le plan pharmaceutique - telle qu'une solution saline ou isotonique - ou à l'aide d'un ou plusieurs excipients tels que un diluant, un liant, un stabilisant, un émulsifiant, un adjuvant etc. A titre alternatif, l'agent chimique peut être incorporé dans l'alimentation ou dans la boisson d'hydratation de l'animal.

Selon un aspect supplémentaire, la présente invention a également pour objet un kit pour la préparation d'un modèle chimique, ledit kit comprenant :
- un agent chimique tel que décrit précédemment, de préférence choisie parmi les composés de formule (I) ou (Ia), leurs sels acceptables sur le plan pharmaceutique et combinaisons, et
- un système cellulaire destiné à être mis en contact avec le composé pour obtenir ledit modèle chimique.

L'agent chimique peut être le pyridinium de furosémide ou l'un de ses sels acceptables sur le plan pharmaceutique. Dans certains modes de réalisation, le système cellulaire est une culture primaire neuronale, d'une lignée cellulaire, de préférence d'origine neuronale, par exemple une lignée de neuroblastome, ou encore un tissu nerveux, de préférence cérébral. De préférence, le système cellulaire est une lignée cellulaire présentant une ou plusieurs caractéristiques biochimiques des neurones dopaminergiques. Il peut s'agir d'une des lignées cellulaires suivantes : SH-SY5Y, SK-N-MC ou PC12.

Dans d'autres modes de réalisation, le système cellulaire est choisi parmi un nématode tel que C. *elegans* ou un poisson, de préférence le poisson zèbre, à l'état adulte ou à l'état de larve.

Le kit peut comprendre également un ou plusieurs consommables tels qu'un milieu de culture, une solution tampon, une microplaque ou encore une notice explicative.

Un objet supplémentaire de la présente invention est l'utilisation de l'agent chimique tel que défini ci-avant pour induire chez un mammifère non-humain un ou plusieurs troubles associés à la maladie de Parkinson. Ces symptômes sont, de préférence, choisis parmi le groupe constitué par une rigidité musculaire, un tremblement au repos, une instabilité posturale, une akinésie, une accumulation intracellulaire d'alpha-synucléine, de préférence sous la forme de corps de Lewy au niveau du cytoplasme de neurones dopaminergiques du stratium et/ou sous forme phosphorylée une diminution de l'activité mitochondriale, en particulier du complexe I, et une dégénérescence des neurones dopaminergiques de la *substantia nigra.* Ces troubles sont induits chez l'animal afin d'étudier les mécanismes biologiques impliqués dans le développement de la maladie de Parkinson ou pour évaluer l'efficacité thérapeutique de médicaments-candidats destinés au traitement ou à la prévention de la maladie de Parkinson.

Au sens de l'invention, le terme « traitement » désigne le fait de ralentir ou de bloquer le développement d'une maladie ou des symptômes associés, ou encore le fait de guérir de ladite maladie. La prévention d'une maladie désigne, quant à elle, le fait de diminuer le risque de l'apparition d'une maladie au sein d'un groupe d'individus donnés.

### Modèle chimique d'une maladie neurodégénérative selon l'invention

Un objet supplémentaire selon la présente invention est le modèle chimique obtenu selon la méthode de préparation décrite ci-avant. Le modèle chimique selon l'invention se distingue du système cellulaire utilisé pour son obtention en ce qu'il présente une ou plusieurs caractéristiques non-observées dans ledit système cellulaire de départ.

Lorsque le modèle chimique est cellulaire, ledit modèle chimique est caractérisé en ce qu'il présente une ou plusieurs fonctions biochimiques augmentées ou diminuées par rapport au système cellulaire de départ. A titre d'exemple, le modèle chimique cellulaire selon l'invention peut présenter une activité caspase, en particulier caspase 3 ou 9, augmentée, et/ou une diminution de l'activité mitochondriale, en particulier du complexe I, et/ou une augmentation de la production et de l'accumulation intracellulaire d'alpha-synucléine par rapport au système cellulaire de départ.

De préférence, le modèle chimique cellulaire selon l'invention présente ces trois caractéristiques.

On considère qu'une fonction biochimique du modèle chimique cellulaire est distincte de celle du système cellulaire de départ, si on observe une variation quantitative pour cette fonction d'au moins 10%, de préférence d'au moins 20%, voire d'au moins 40%. La quantification des fonctions biologiques dans le modèle chimique et dans le système cellulaire de départ pourra être effectuée par l'une quelconque des méthodes connues de l'homme du métier. On pourra, en particulier, se référer aux exemples de la présente demande de brevet.

Lorsque le modèle chimique est un modèle animal, ledit modèle chimique peut se distinguer du système cellulaire de départ en ce qu'il présente une ou plusieurs caractéristiques histologiques ou anatomiques et/ou un phénotype comportemental distincts de ceux observés pour le système cellulaire de départ. Le modèle chimique animal selon l'invention peut ainsi présenter une dégénérescence d'un ou plusieurs neurones, de préférence dopaminergiques, et/ou une agrégation cytoplasmique d'alpha-synucléine. Le modèle animal peut, de plus ou à titre alternatif, présenter un phénotype comportemental distinct, en particulier un trouble moteur tel qu'une diminution de la capacité de locomotion, c'est-à-dire de la mobilité ou encore un trouble de l'éveil ou du sommeil, par exemple une augmentation du nombre de phases de sommeil paradoxal.

Par exemple, il est attendu que le modèle chimique obtenu à partir de C. *elegans* se caractérise, d'un point de vue comportemental, par des troubles de la mobilité, en particulier des mouvements lents et irréguliers accompagnés de secousses et, d'un point de vue anatomique, par une dégénérescence neuronale majoritairement dopaminergique.

A titre d'exemple supplémentaire, il est attendu que le modèle chimique obtenu à partir de larves de poisson zèbre présente des troubles de la mobilité, en particulier une diminution de la vitesse de nage, et une neurodégénérescence dopaminergique.

Dans le mode de réalisation particulier où le modèle cellulaire est un mammifère, ledit modèle peut présenter une lésion dopaminergique de la *substantia nigra* et/ou une accumulation intracellulaire d'alpha-synucléine, éventuellement phosphorylée et/ou sous la forme de corps de Lewy. Il peut également se caractériser par un ou plusieurs troubles moteurs choisis parmi le groupe constitué par une rigidité musculaire, une baisse de mobilité, en particulier une akinésie, une instabilité posturale ou des tremblements au repos. Il peut, en outre, présenter un ou plusieurs symptômes non-moteurs tels que des troubles du sommeil ou de l'olfaction.

A titre d'exemple supplémentaire, le modèle chimique obtenu chez la souris présente, lors de l'examen histologique post mortem, une dimunition de l'expression de la tyrosine hydoxylase et une accumulation d'alpha-synucléine phosphorylée au niveau du stratium. A toute fin utile, on rappelle, qu'à la connaissance du Demandeur, aucun cas spontané de maladie de Parkinson n'a été rapporté pour un animal non-humain.

### Utilisations des modèles chimiques selon l'invention et kits de mise en oeuvre

Les modèles chimiques selon l'invention trouvent de nombreuses applications en recherche fondamentale, en particulier pour l'étude des différents stades, en particulier des stades précoces de la maladie de Parkinson. Ces modèles chimiques trouvent également des applications en recherche pharmaceutique, en particulier pour l'identification ou l'évaluation de nouveaux candidat-médicaments pour le traitement ou la prévention des maladies neurodégénératives, en particulier des synucléinopathies, y compris la maladie de Parkinson.

Ainsi, la présente invention a également pour objet l'utilisation d'un modèle chimique tel que décrit précédemment dans un test de criblage de composés destinés au traitement ou à la prévention d'une maladie neurodégénérative.

L'invention a également pour objet l'utilisation d'un modèle chimique tel que décrit précédemment pour évaluer l'efficacité d'un principe actif dans le traitement ou la prévention d'une maladie neurodégénérative, le modèle chimique étant de préférence un mammifère non humain.

Dans certains modes de réalisation, la maladie neurodégénérative est une synucléinopathie, de préférence la maladie de Parkinson. Il va de soi que le modèle chimique utilisé correspond à la pathologie visée par le test de criblage ou le candidat médicament. A titre d'exemple, si l'on recherche des composés destinés au traitement ou à la prévention de la maladie de Parkinson, on utilisera un modèle chimique correspondant à la maladie de Parkinson.

La présente invention a, de plus, pour objet un procédé pour évaluer, cribler ou sélectionner un composé destiné au traitement ou la prévention d'une synucléinopathie, de préférence la maladie de Parkinson, ledit procédé comprenant :
a) la mise en contact d'un système cellulaire avec l'agent chimique tel que décrit précédemment,
b) la mise en contact dudit système cellulaire avec le composé à tester, et
c) la détection ou la quantification d'un marqueur caractéristique de la synucléinopathie,
l'étape b) pouvant être mise en oeuvre simultanément, avant ou après l'étape a) ou chevaucher l'étape a).

L'étape a) est mise en oeuvre dans des conditions permettant d'obtenir le modèle chimique selon l'invention, si le composé à tester n'est pas rajouté (c'est-à-dire en l'absence d'une mise en contacte préalable ou simultanée avec le composé à tester).

L'étape c) est mise en oeuvre en vue de déterminer l'efficacité potentielle du composé. On entend par « efficacité », la capacité du composé à moduler le marqueur caractéristique de la synucléinopathie par rapport à une expérience témoin ou à des valeurs de référence préalablement obtenues.

Ainsi, le procédé selon l'invention peut comprendre les étapes consistant à :
a) Mettre en contact un système cellulaire avec l'agent chimique tel que décrit précédemment de manière à obtenir un modèle chimique tel que défini précédemment,
b) Mettre en contact ledit système cellulaire avec le composé à tester, et
c) Détecter ou quantifier un marqueur caractéristique de la synucléinopathie permettant de déterminer l'efficacité du composé à tester,
l'étape b) pouvant être mis en oeuvre simultanément, avant ou après l'étape a) ou chevaucher l'étape a).

Le système cellulaire peut être choisi parmi le groupe constitué par une cellule, une culture cellulaire, un tissu ou un animal non-humain, comme précédemment décrit.

Les étapes de « mises en contact » a) et b) peuvent être réalisées par incubation ou par administration, en fonction du système cellulaire choisi.

De préférence, l'agent chimique est choisi parmi les composés de formule (I), leurs sels acceptables sur le plan pharmaceutique, et les combinaisons de ceux-ci. De manière encore plus préférée, l'agent chimique est le pyridinium de furosémide ou l'un de ses sels acceptables sur le plan pharmaceutique.

Le procédé selon l'invention peut comprendre une ou des étapes supplémentaires, en particulier des étapes de lavage ou des étapes de traitement du système cellulaire.

Dans certains modes de réalisation, un groupe de contrôle, c'est-à-dire un système cellulaire qui subit l'ensemble des étapes ci-dessus décrites mais qui n'est pas mis en contact avec le composé à tester, est utilisé.

Ainsi dans certains modes de réalisation, le procédé selon l'invention comprend :
a) la mise en contact du système cellulaire avec l'agent chimique tel que décrit précédemment,
b) la mise en contact dudit système cellulaire avec le composé à tester, cette mise en contact pouvant être simultanée, chevauchée, précédée ou postérieure à l'étape a),
c) la détection ou la quantification d'un marqueur caractéristique de la synucléinopathie dans ledit système cellulaire, et
d) la comparaison du résultat obtenu à l'étape c) avec la détection ou la quantification dudit marqueur dans un système cellulaire témoin obtenu par mise en contact avec l'agent chimique, en l'absence du composé à tester.

La comparaison effectuée à l'étape d) permet de sélectionner le composé testé en tant que candidat-médicament, ou de l'écarter. Des exemples de mise en oeuvre sont fournis plus loin dans la description.

De préférence, l'agent chimique est le pyridinium de furosémide.

On entend par « marqueur caractéristique d'une maladie » une modification biochimique, histologique, anatomique ou comportementale associée à la maladie, que l'on observe chez l'individu atteint par la maladie ou chez un modèle chimique ou génétique de cette maladie par comparaison avec un individu sain ou un système cellulaire sain de départ.

Dans certains modes de réalisation du procédé selon l'invention, le système cellulaire est une cellule, une culture cellulaire ou un tissu nerveux, de préférence cérébral. De préférence, le système cellulaire est une lignée cellulaire présentant une ou plusieurs caractéristiques biochimiques des neurones dopaminergiques. Par exemple, la lignée cellulaire est choisie parmi les lignées SH-SY5Y, SK-N-MC et PC12.

Dans ce mode de réalisation, le marqueur caractéristique de la synucléinopathie est de préférence choisi parmi le groupe constitué par l'accumulation intracellulaire d'une synucléine, de préférence d'α-synucléine, un marqueur de la mort cellulaire par apoptose, de préférence une augmentation d'une activité caspase, telle que l'activité caspase 3 et/ou 9, une diminution du taux de survie cellulaire, une diminution de l'activité de la chaîne respiratoire mitochondriale, en particulier une diminution de l'activité du complexe I mitochondrial et leurs combinaisons.

La quantification de ces marqueurs peut être réalisée selon des techniques bien connues de l'homme du métier. On considère qu'il y a augmentation ou diminution d'un marqueur lorsque l'on observe une variation d'au moins ± 10%, de préférence d'au moins ± 20%, par rapport à la valeur du marqueur quantifié chez le système cellulaire de départ (c'est-à-dire non-exposé à l'agent chimique).

A titre d'exemple, le taux de survie cellulaire peut être quantifié par le test de viabilité au MTT. Les activités caspase peuvent être mesurées à partir d'un test basé sur la capacité de chaque caspase à cliver spécifiquement un substrat fluorescent (Yue et al. Cell Death Differ. 2009;16(5):770-81.). La quantification de l'accumulation intracellulaire d'alpha-synucléine peut être réalisée au moyen d'anticorps anti-synucléine marqués ou couplés à une enzyme telle que la horseradish peroxidase, par exemple par western blot comme décrit dans les exemples ou encore par un test ELISA.

Une diminution de l'activité de la chaîne respiratoire mitochondriale peut être quantifiée en mesurant la consommation d'oxygène des mitochondries par polarographie et en évaluant le couplage de la phosphorylation oxydative en présence de substrats du complexe I ou du complexe II, ou encore en mesurant l'activité du complexe I par spectrophotométrie ou la production d'espèce réactive de l'oxygène. Des méthodes pour mesurer la diminution de l'activité mitochondriale sont fournies, par exemple, dans Morin et al. (Neuroscience. 2002;115(2):415-24) et Cormier et al. (rain Res. 2001 May 4;900(1):72-9).

Dans d'autres modes de réalisation, le système cellulaire est un animal non-humain. Le marqueur caractéristique de la synucléinopathie est choisi parmi le groupe constitué par un trouble moteur, en particulier un trouble de la mobilité, un trouble de l'éveil ou du sommeil, une dégénérescence des neurones dopaminergiques, l'accumulation intracellulaire d'alpha-synucléine au niveau d'un neurone dopaminergique et leurs combinaisons.

Si l'on souhaite évaluer un grand nombre de composés, par exemple dans le cadre d'un test de criblage à haut-débit, on utilise de préférence un système cellulaire choisi parmi le groupe constitué par une cellule, une culture cellulaire, un tissu ou un animal de petite taille tel que le poisson zèbre ou de *C*. *elegans.*

L'ordre des étapes de mise en contact a) et b) dépendra du type de composés que l'on souhaite identifier.

Si l'on recherche un composé à activité préventive ou capable de ralentir le développement de la maladie, on peut, dans un premier temps, incuber le composé à tester avec le système cellulaire pendant une ou plusieurs minutes, voire plusieurs heures, puis rajouter dans le milieu l'agent chimique (e.g. le pyridinium de furosémide). A titre alternatif, les étapes a) et b) peuvent être simultanées.

Si l'on recherche un composé à activité curative, on peut mettre en contact le système cellulaire avec l'agent chimique (e.g. pyridinium de furosémide), par exemple pendant une durée de 24 h à 48h, puis dans un second temps, on rajoute dans le milieu le composé à tester. Le système cellulaire peut être lavé avant l'introduction du composé à tester : les étapes a) et b) sont donc, dans ce cas, successives.

Pour la mise en oeuvre des étapes a) et b) par incubation, la concentration de l'agent chimique (e.g. pyridinium de furosémide) et celle du composé à tester sont généralement choisies dans une gamme allant de 1.10⁻¹⁰ à 1 mol.L⁻¹, de préférence de 1.10⁻⁸ à 1.10⁻² mol.1⁻¹.

Dans un mode de réalisation particulier, le système cellulaire est un nématode, en particulier *C*. *elegans.* Dans ce cas, l'homme du métier pourra se référer l'article de Braungart et al. (Neurodegenerative Disease, 2004, 1 :175-183) pour la mise en oeuvre du procédé selon l'invention.

Dans ce mode de réalisation, le marqueur caractéristique de la synucléinopathie peut être choisi entre une diminution de la mobilité ou la dégénérescence des neurones dopaminergiques. Des méthodes de quantification pour ces deux marqueurs sont décrites dans Braungart et al. (voir *supra*) qui est incorporé ici par référence.

La quantification de la dégénérescence des neurones dopaminergiques peut être réalisée en utilisant une souche de *C*. *elegans* exprimant spécifiquement la protéine GFP au niveau des neurones dopaminergiques, par exemple la souche cat-2::GFP. La quantification de la dégénérescence dopaminergique est alors réalisée par mesure de la fluorescence à une longueur d'onde d'excitation adaptée.

A titre d'exemple, le procédé selon l'invention peut être mis en oeuvre en microplaque en utilisant la souche de *C*. *elegans* cat-2 ::GFP décrite par Lints et al. (Development 1999,126 :5819-5831). Dans chaque puits de la microplaque, on distribue un nombre donné de nématodes en suspension dans un milieu de culture adapté. Les puits de la microplaque sont répartis en quatre groupes. Dans le groupe 1, les nématodes sont simultanément incubés avec le pyridinium de furosémide et le composé à tester. Dans le groupe 2, les nématodes sont incubés avec le pyridinium de furosémide uniquement. Dans le groupe 3, les nématodes ne sont mis en contact ni avec le pyridinium, ni avec le composé à tester. Enfin, dans le groupe 4, les nématodes sont incubés seulement avec le composé à tester. Les quatre groupes sont incubés pendant une durée suffisamment longues, typiquement pendant plusieurs heures voire quelques jours. Après incubation, la mobilité et/ou l'étendue de la dégénérescence neuronale sont quantifiées Les valeurs obtenues pour le groupe 3 permettent de normaliser au sein d'une même microplaque les résultats obtenus pour les groupes 1 et 2. L'efficacité du composé à tester est déterminée en comparant les résultats du groupe 2 avec ceux obtenus pour le groupe 1. L'innocuité du composé à tester peut être vérifiée en comparant les résultats obtenus pour le groupe 4 à ceux obtenus pour le groupe 3.

On peut considérer que le composé testé est efficace, et donc à sélectionner, s'il induit, chez les individus du groupe 1, une augmentation d'au moins 10% de la mobilité moyenne et/ou une augmentation d'au moins 10% de la fluorescence par rapport aux individus du groupe 2.

Dans d'autres modes de réalisation, le système cellulaire est un poisson zèbre, soit à l'état adulte, soit à l'état de larve. L'utilisation du poisson zèbre dans des tests de criblage est bien connue de l'homme du métier et est décrite par exemple dans l'article de revue de Rubinstein (Expert Opin. Drug Metab. Toxicol, 2006, 231-240). On peut par exemple utiliser une lignée de poisson zèbre exprimant une alpha-synucléine couplée à une sonde GFP ou une lignée possédant des mutations au niveau des gènes couramment impliqués dans la maladie de Parkinson (PARKIN, LRRK2, DJ-1...).

Dans un mode de réalisation supplémentaire, le système cellulaire est un mammifère non-humain tel que décrit précédemment. De préférence, il s'agit d'un primate ou d'un rongeur. Comme dans le cas des systèmes cellulaires de petite taille, l'ordre des étapes a) et b) du procédé selon l'invention dépend du type de composés que l'on souhaite identifier. Par ailleurs, selon le schéma d'administration choisi, les étapes a) et b) peuvent être répétées plusieurs fois.

Si l'on recherche un composé à action préventive ou permettant de ralentir le développement de la maladie, le composé à tester peut être administré à l'animal avant (typiquement quelques heures avant) l'agent chimique (e.g. pyridinium de furosémide). Si l'on recherche un composé à activité curative, le composé à tester pourra être administré à l'animal après l'agent chimique ou simultanément.

Le composé à tester et l'agent chimique peuvent être administrés par toute voie, de manière préférée, par voie orale, intrapéritonéale, intranasale, intraveineuse, sous-cutanée, intramusculaire et intracrânienne. Les voies d'administration de l'agent chimique et du composé à tester peuvent être identiques ou différentes.

Les doses à administrer dépendent de l'animal utilisé, en particulier, de son poids, de la voie d'administration choisie, et de sa sensibilité au pyridinium de furosémide et au composé testé. L'homme du métier saura, par des tests de routine, déterminer le dosage et le schéma d'administration adéquate. Typiquement, la dose à administrer peut varier de 200 ng/kg à 100 mg/kg. Le schéma d'administration peut comprendre l'administration d'une dose unique ou l'administration de plusieurs doses sur plusieurs heures, plusieurs jours ou plusieurs semaines de chacun des composés.

Dans ce mode de réalisation, le marqueur quantifié ou détecté dans le procédé selon l'invention peut être une lésion anatomique, telle qu'une dégénérescence des neurones dopaminergiques de la *substantia nigra* et l'apparition d'agrégation d'alpha-synucléines par exemple sous la forme de neurites de Lewy et/ou de corps de Lewy. La lésion anatomique peut être détectée par imagerie *in vivo,* par exemple comme décrit dans la demande PCT WO2012/065045, ou par analyse histologique post-mortem.

Le marqueur de la synucléinopathie peut être également un trouble moteur tel qu'une rigidité musculaire, une baisse de mobilité, en particulier une akinésie, une instabilité posturale ou des tremblements. Il est à noter que le trouble moteur induit chez le modèle chimique peut dépendre de l'animal utilisé. L'homme du métier pourra se référer aux travaux décrits dans l'état de la technique pour déterminer les troubles moteurs à étudier en fonction de l'animal choisi et la manière de les quantifier.

Un composé sera considéré comme actif s'il améliore ou prévient l'apparition d'un trouble moteur induit par le pyridinium de furosémide et/ou s'il prévient la lésion neurologique. Selon un aspect supplémentaire, la présente invention a pour objet un kit pour la mise en oeuvre d'un procédé de criblage tel que décrit ci-avant, ledit kit comprenant :
- un agent chimique, de préférence choisi parmi le groupe de composés de formule (I), leurs sels acceptables sur le plan pharmaceutique et leurs combinaisons
- un système cellulaire tel que décrit précédemment.

Dans certains modes de réalisation, le système cellulaire est une culture cellulaire neuronale, d'une lignée cellulaire, de préférence d'origine neuronale ou un tissu nerveux, de préférence cérébral.

Le système cellulaire peut être, par exemple une lignée cellulaire présentant une ou plusieurs caractéristiques biochimiques des neurones dopaminergiques. Par exemple, la lignée cellulaire peut être choisie parmi les lignées SH-SY5Y, SK-N-MC et PC12.

Dans d'autres modes de réalisation, le système cellulaire est un animal non-humain de préférence, un nématode tel que *C*. *elegans* ou un poisson, tel que le poisson zèbre, à l'état adulte ou à l'état de larve.

Avantageusement, le système cellulaire est choisi parmi le groupe constitué par une lignée cellulaire, un nématode, tel que *C*. *elegans,* et un poisson, tel que le poisson-zèbre, le poisson et le nématode pouvant être à l'état de larve ou à l'état adulte.

Le kit selon l'invention peut, en option, comprendre un moyen pour la détection ou la quantification d'un marqueur caractéristique de la synucléinopathie, par exemple un anticorps anti-synucléine.

Le kit peut comprendre également un ou plusieurs consommables tels qu'un milieu de culture, une solution tampon, une microplaque ou des molécules de référence ou étalon pour la mise en oeuvre du procédé de criblage. Enfin, le kit peut comprendre une notice explicative de mise en oeuvre du test de criblage.

Par exemple, le kit peut comprendre en tant que molécules de références, un agent antioxydant capable de prévenir l'effet de l'agent chimique, tel que l'alpha-tocophérol.

### Test d'évaluation de la neurotoxicité d'un composé

De manière surprenante, le Demandeur a montré que le pyridinium de furosémide présentait une neurotoxicité cellulaire décelable après une longue période d'incubation d'environ 96 h. Un tel résultat est particulièrement surprenant au regard des tests de toxicité utilisés à l'heure actuelle. A la connaissance du Demandeur, ces tests prévoient des durées d'incubation de la molécule à tester avec le modèle cellulaire de référence d'au plus de 48 h. Le pyridinium de furosémide n'aurait donc pas été identifié comme neurotoxique par la mise en oeuvre de ces tests.

Le pyridinium de furosémide ainsi que ses dérivés, métabolites et prodrogues tels que décrits précédemment, en particulier les composés de formule (I) ou (la), peuvent donc trouver une utilisation en tant que composé témoin, ou encore en tant que composé étalon, pour la mise en oeuvre d'un test visant à évaluer la toxicité de composés, par exemple de polluants environnementaux.

L'invention a donc également pour objet l'utilisation d'un agent chimique tel que défini ci-avant en tant que témoin positif dans un test d'évaluation de la toxicité, de préférence la neurotoxicité, d'un composé.

Le test d'évaluation comprend, de préférence, l'incubation d'un système cellulaire avec le composé à tester pendant une durée supérieure à 48h, de préférence d'au moins 60h.

Un objet supplémentaire de la présente invention est une méthode pour évaluer la toxicité, de préférence, la neurotoxicité d'un composé, ladite méthode comprenant les étapes :
a) réaliser en parallèle les incubations suivantes, pendant une durée d'incubation supérieure à 48 h, de préférence d'au moins 60 h :
   i. l'incubation d'un système cellulaire avec une solution d'incubation comprenant le composé à tester,
   ii. l'incubation d'un système cellulaire avec une solution d'incubation comprenant une molécule témoin, ladite molécule témoin étant l'agent chimique tel que décrit ci-avant, et
   iii. l'incubation d'un système cellulaire avec une solution d'incubation exempte du composé à tester et de la molécule témoin.
b) quantifier pour chaque système incubé à l'étape a) un marqueur spécifique de la neurotoxicité,
c) comparer les valeurs obtenues à l'étape b) entre elles de manière à déterminer la toxicité du composé.

Les incubations ii) et iii) correspondent aux expériences témoin positif et négatif, respectivement. Il va de soi que les systèmes cellulaires utilisés aux étapes i), ii), et iii) sont identiques. A titre d'exemple, pour la mise en oeuvre des étapes i), ii), et iii) ont peut utiliser des larves de poisson zèbre. Le système cellulaire utilisé dans la méthode selon l'invention est de préférence une culture cellulaire neuronale, d'une lignée cellulaire d'origine neuronale ou un tissu nerveux, de préférence cérébral. De préférence, le système cellulaire est une lignée cellulaire, de préférence d'origine neuronale et/ou présentant une ou plusieurs caractéristiques biochimiques des neurones dopaminergiques. Par exemple, la lignée cellulaire est choisie parmi les lignées SH-SY5Y, SK-N-MC et PC12.

Dans d'autres modes de réalisation, le système cellulaire est choisi parmi un animal non-humain de préférence, un nématode tel que *C*. *elegans* ou un poisson, tel que le poisson zèbre, à l'état adulte ou à l'état de larve.

Une durée d'incubation supérieure à 48 h englobe une durée d'incubation d'au moins 54 h, d'au moins à 60h, d'au moins 66 h, d'au moins 72h, d'au moins à 78h, d'au moins à 84 h, d'au moins 90h, d'au moins 96h.

A titre d'exemple, l'étape c) peut être réalisée comme suit :
Dans un premier temps, les valeurs obtenues pour les incubations ii) et iii) sont comparées. Si une variation de moins de X% est obtenue pour ces valeurs, on considère que le test n'a pas été réalisé correctement et doit être réalisé, éventuellement, en augmentant la durée d'incubation. En revanche, si on observe une variation de plus de X% entre les valeurs obtenues pour les incubations ii) et iii), on considère que le test a été correctement réalisé et que l'on peut comparer les valeurs obtenues pour les incubations i) et iii). Si on observe une variation de plus de Y% des valeurs i) et iii), on peut conclure que le composé testé est toxique.

Les valeurs seuil de variation X et Y dépendent du marqueur quantifié. En général, ces valeurs sont comprises entre 5 et 50.

Sans vouloir être lié par une quelconque théorie, le Demandeur pense que la méthode d'évaluation de la toxicité selon l'invention, de par l'utilisation du pyridinium de furosémide ou de l'un de ses dérivés en tant que témoin positif et l'utilisation d'un temps d'incubation long, permettra de discriminer des composés neurotoxiques, qui ne seraient pas identifiés comme tels par la mise en oeuvre des tests actuellement en vigueur. Ce test peut trouver une application dans l'identification du potentiel neurotoxique des polluants environnementaux - tels que les herbicides, pesticides, résidus médicamenteux - auxquels l'homme est généralement exposé de manière chronique, sur une longue durée et à faible dose.

L'invention se rapporte également à un kit pour l'évaluation de la toxicité, de préférence la neurotoxicité, d'un composé, ledit test comprenant:
- le pyridinium de furosémide ou l'un de ses sels, prodrogues, métabolites ou dérivés, de préférence un composé de formule (I) ou (la) en tant que composé témoin,
- un système cellulaire,
- en option, un ou plusieurs consommables tels qu'un milieu de culture, une solution tampon, ou une microplaque
- en option, une note explicative décrivant la mise en oeuvre du test, et
- en option, un ou plusieurs moyens pour la détection ou la quantification d'un marqueur de toxicité.

Dans les utilisations, méthode et kit pour l'évaluation de la toxicité d'un composé ci-dessus décrit, le système cellulaire est de préférence une culture cellulaire neuronale, une lignée cellulaire, de préférence d'origine neuronale, par exemple une lignée de neuroblastome, ou un tissu nerveux, de préférence cérébral. De préférence, le système cellulaire est choisi parmi les lignées cellulaires SH-SY5Y, SK-N-MC et PC12.

Dans d'autres modes de réalisation, le système cellulaire est choisi parmi un animal non-humain de préférence, un nématode tel que *C*. *elegans* ou un poisson, tel que le poisson zèbre, à l'état adulte ou à l'état de larve.

Les marqueurs de toxicité comprennent, sans y être limités, une augmentation d'une activité caspase, une augmentation du taux de mort cellulaire, une diminution de l'activité mitochondriale, une augmentation de la production d'espèces réactives de l'oxygène, une augmentation de la quantité intracellulaire d'α-synucléine, une diminution de la mobilité et une dégénérescence de neurones dopaminergiques.

Le ou les marqueurs de toxicité à quantifier dépendront du système cellulaire choisi. A titre d'exemple, si l'on utilise *C. elegans* ou une larve de poisson zèbre en tant que système cellulaire, on étudiera l'effet du composé à tester sur la mobilité, la dégénérescence des neurones dopaminergiques et/ou l'accumulation d'alpha-synucléine. A titre alternatif, si l'on utilise la lignée cellulaire SH-SY5Y, la toxicité du composé pourra être déterminée en quantifiant une activité caspase, telle qu'une activité caspase 3 et/ou 9, la quantité intracellulaire d'alpha-synucléine, l'activité du complexe I de la chaine respiratoire mitochondriale et/ou le taux de survie cellulaire à l'issue de l'incubation.

### Composés de formule (I)

La description a également pour objet un composé de formule (I): 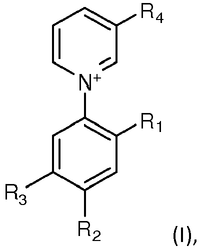 dans laquelle,
- R₁ représente un atome d'hydrogène, un atome d'halogène, un alkyle en C₁-C₄,-OH, -CN, -C(=O)NH₂, -CF₃, -C(=O)O⁻, ou -C(=O)O-R₅ avec R₅ représente -H ou un alkyle en C₁-C₄.
- R₂ est -SO₂NH₂, un alkyle en C₁-C₄, un atome d'hydrogène, un atome d'halogène, - OH, -CN, -C(=O)NH₂, -CF₃, ou -C(=O)OR₆ avec R₆ représente H ou un alkyle en C₁-C₄
- R₃ est H, un atome d'halogène, un alkyle en C₁-C₄. -OH, -CN, -C(=O)NH₂, -CF₃, ou -C(=O)OR₇ avec R₇ représente H ou un alkyle en C₁-C₄. et
- -R₄ représente un atome d'hydrogène, -OH ou -OCH₃,
ou un sel pharmaceutiquement acceptable de celui-ci.

Un atome d'halogène englobe Br, Cl, I ou F, de préférence Br et Cl. Un groupe alkyle en C₁-C₄ englobe les groupes méthyle, éthyle, propyle et butyle.

Il va de soi que l'agent chimique comprend le ou les contre-anions ou contre-cations nécessaires en fonction de l'état d'ionisation des goupes R₁, R₂, R₃ et R₄.

Dans certains modes de réalisation de l'invention, le composé de formule (I) est tel que
- R₁ est -H, un atome d'halogène, -CN, -C(=O)NH₂, -CF₃, -C(=O)O⁻, ou -C(=O)OR₅ avec R₅ représente -H ou un alkyle en C₁-C₄,
- R₂ est -SO₂NH₂, -C(=O)NH₂, un atome d'halogène, -H, -CN, ou CF₃,
- R₃ est -H, un atome d'halogène, -CN, -CONH₂, -CF₃, -C(=O)O⁻, ou -C(=O)OR₇ avec R₇ représente -H ou un alkyle en C₁-C₄, et
- R₄ est -OH, -OMe ou -H sous réserve que R₂ ne soit pas -SO₂NH₂, quand R₁ représente -C(=O)O⁻ ou -(C=O)OH, R₃ est -Cl et R₄ est OH.

Dans un autre mode de réalisation de l'invention, le composé de formule (I) est tel que
- R₁ est -H, un atome d'halogène, -CN, -C(=O)NH₂, -CF₃, -C(=O)O⁻, ou -C(=O)OR₅ avec R₅ représente -H ou un alkyle en C₁-C₄,
- R₂ est -SO₂NH₂, -C(=O)NH₂, un atome d'halogène, -H, -CN, ou CF₃,
- R₃ est -H, un atome d'halogène, -CN, -CONH₂, ou -CF₃, et
- R₄ est -OH, -OMe ou -H sous réserve que R₂ ne soit pas -SO₂NH₂, quand R₁ représente -C(=O)O⁻ ou -(C=O)OH, R₃ est -Cl et R₄ est OH.

Dans un mode de réalisation supplémentaire, l'agent chimique est un composé de formule (I) dans laquelle :
- R₁ représente un atome d'hydrogène, -C(=O)O⁻, ou -C(=O)O-R₅ où R₅ est H ou un groupe alkyle en C_{1-C4,},
- R₂ est -SO₂NH₂, un atome d'hydrogène, ou un atome d'halogène,
- R₃ est H ou un atome d'halogène, et
- R₄ est -OH ou -H, sous réserve que R₂ ne soit pas -SO₂NH₂, quand R₁ représente-C(=O)O⁻ ou -(C=O)OH, R₃ est -Cl et R₄ est OH
ou un sel acceptable sur le plan pharmaceutique de celui-ci.

Dans certains modes de réalisation, R₄ est -OH. La description a de plus pour objet un composé de formule (la) 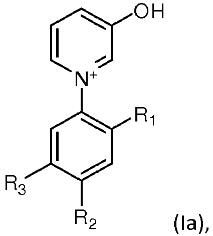 Dans laquelle,
- R₁ représente un atome d'hydrogène, -C(=O)O⁻, ou -C(=O)O-R₅ où R₅ est H ou un groupe alkyle en C_{1-C4,},
- R₂ est -SO₂NH₂, un atome d'hydrogène, ou un atome d'halogène,
- R₃ est H ou un atome d'halogène,
- sous réserve que R₂ ne soit pas -SO₂NH₂, quand R₁ représente -C(=O)O⁻ ou-(C=O)OH, et R₃ est -Cl,
ou un sel acceptable sur le plan pharmaceutique de celui-ci.

Les exemples ci-après ont pour but d'illustrer plus pleinement l'invention sans pour autant en limiter la portée.

### Exemples

### Partie A : Chimie

Le MPP+ est acheté sous forme de sel d'iodure chez Sigma-Aldrich (CAS : 36913-39-0). Le furosémide est acheté chez TCI Europe N.V. (Numéro CAS : 54-31-9)

### 1- Synthèse du pyridinium de furosémide

Le pyridinium de furosémide a été synthétisé à partir du furosémide comme décrit dans Laurencé et al, Tetrahedron 2011.

### - Synthèse de l'acide 4-Chloro-2-[(2,5-diméthoxy-2,5-dihydro-furan-2-ylméthyl)-amino]-5-sulfamoyl-benzoique (Composé (1))

Dans une cellule en verre indivisée équipée de deux électrodes en carbone, le furosémide (250 mg, 0.75 mmol), le bromure d'ammonium (61 mg, 0.6 mmol), et le tétrafluoroborate de tetraéthylammonium (81 mg, 0,4 mmol) ont été dissous dans le méthanol (10 mL). Le milieu réactionnel a été soumis à une agitation magnétique et soumis à un courant constant de 30mA jusqu'à ce que 2.5 F/mol ait été consommé. La solution a été transférée dans un ballon et le méthanol a été évaporé sous pression réduite. Le résidu obtenu a été resuspendu dans de l'acétate d'éthyle et les sels inorganiques ont été éliminés par filtration. Après évaporation de l'acétate d'éthyle, un solide beige contenant deux diastéréoisomères selon un ratio 50 :50 a été obtenu (268 mg, 91%).
¹H NMR (400 MHz, acetone-d6): δ = 8,55 (s, 2H), 7,07 (s, 1H), 7,05 (s, 1H), 6,22- 6,15 (m,2H), 6,05 (d, J=13,4 Hz,1H), 6,03 (d, J=13,5 Hz,1H), 5,79 (br s,1H), 5,52 (br s,1H), 3,72-3,61 (m, 2H); 3,57-3,47 (m, 2H); 3,45 (s, 3H), 3,31 (s, 3H), 3,20 (s, 3H), 3,12 (s, 3H); ¹³C NMR (acetone-d6): δ = 169,3, 169,1, 154,8, 154,5, 138,0, 137,8, 135,0, 135,0, 134,2, 134,2, 132,4, 132,1, 127,4, 127,2, 114,9, 114,7, 114,6, 114,0, 109,9, 108,6, 108,5, 56,4, 56,0, 50,7, 50,4, 50,1, 49,9; IR: 3257, 1674, 1595, 1503,1455, 1331, 1229,1162, 1043, 1012, 957, 899, 832, 799, 686, 626, 579 cm⁻¹ ;
HRMS m/z calculé pour C₁₄H₁₈ClN₂O₇S [M+H]+: 393,0523.Trouvé: 393,0445.

### - Synthèse du pyridinium de furosémide

Le pyridinium de furosémide a été obtenu en dissolvant le composé (1) (136 mg, 0,3 mmol) dans 2 mL d'un mélange TFA/MeCN 60/40. Le mélange réactionnel a été soumis à une température de 40°C sous agitation. Le précipité formé a été récupéré par filtration. Un solide blanc de pyridinium de furosémide a été ainsi obtenu.
Point de fusion: 258-260 °C;
¹H NMR (100 MHz, DMSO-d6): δ = 8,78 (br s, 1H), 8,59 (br s, 1H), 8,50 (s, 1H), 8,09 (s, 1H), 8,00e7,87 (m, 4H);
¹³C NMR (100 MHz, DMSO-d6): δ =164,8, 158,0, 143,2, 142,7, 134,9, 134,4, 133,1, 133,1, 131,5, 131,4, 129,8, 127,4; IR: 3096, 1613, 1573, 1494, 1379, 1321, 1252, 1155, 1026, 963, 927, 865, 805, 694, 654, 603, 584 cm⁻¹;
HRMS m/z calculé pour C₁₂H₁₀ClN₂O₅S [M+H]+: 328,9999, Trouvé: 328,9995,
Anal, Calculé pour C₁₂H₉ClN₂O₅S_{$}2H₂O: C 39,51%, H 3,59%, N 7,68%. Trouvé: C 39,47%, H 3,61%, N 7,46%

### 2- Synthèse d'analogues du pyridinium de furosémide

On présente ci-après, à titre d'exemple, la synthèse de deux analogues du pyridinium de furosémide. D'autres analogues ont été préparés selon des méthodes similaires.

### • Préparation d'un analogue de formule I dans lequel R₄ est OH: le chlorure de 1-(4-chlorophenyl)-3-hydroxypyridinium.

***(i) Amination réductrice*** : Dans un ballon, sont introduits 3,00 g (14,45 mmol, 1 éq) de 4-chloro-aniline, 2,4 mL de furaldéhyde distillé (28,9 mmol, 2 éq) et 2,73 g (43,35 mmol, 3 éq) de cyanoborohydrure de sodium dans 50,0 mL d'acétonitrile. Ensuite 396 µL (6,94 mmol, 0,48 éq) d'acide acétique sont ajoutés. Cet ajout est renouvelé 1h après le début de la réaction. Le mélange est chauffé à 40°C et laissé sous agitation pendant une nuitAprès évaporation de l'acétonitrile, le mélange réactionnel est dissous dans 30 ml de tétrahydrofurane puis 8 équivalents de NaHSO₃ à saturation dans l'eau (s = 420 g/L) sont ajoutés, afin d'éliminer le furaldéhyde résiduel. Le mélange est laissé sous agitation vigoureuse à 40°C pendant 4h. Une fois la réaction terminée, la phase organique est extraite au diéthyle éther puis séchée par Na₂SO₄ avant d'être filtrée sur fritté. Après évaporation du solvant, la 4-chloro-N-(furan-2ylméthyl) aniline est purifiée par chromatographie flash sur phase inverse (colonne C18, éluant : méthanol/eau, 2/8 → 8/2) (2,81g, 94%).
***(ii)*** ***Réaction de protection:*** Dans un ballon, sont introduits 1,41 g de 4-chloro-N-(furan-2ylméthyl) aniline (6, 8 mmol, 1 éq) et 1,75 mL de di-tert-butyl dicarbonate (8,16 mmol, 1,2 éq) dans 20 mL de tétrahydrofurane distillé. Le mélange est laissé sous agitation à reflux du tétrahydrofurane (~70°C) pendant 2 jours. A l'issue de la réaction, le produit est récupéré par extraction au dichlorométhane et le di-tert-butyl dicarbonate résiduel est hydrolysé par lavage avec une solution à 0,1 M de HCl. La phase organique est séchée par Na₂SO₄ avant d'être filtrée sur fritté. Après évaporation du solvant, le tert-butyl (furan-2-yméthyl) (4-chloro-phenyl) carbamate est purifié par chromatographie flash sur phase inverse (colonne C18, éluant : méthanol/eau, 2/8 → 8/2) (1,96g, 93%).
***(iii) Oxydation électrochimique et hydrolyse :*** L'électrolyse est réalisée à température ambiante dans une cellule non divisée où l'anode et la cathode sont des plaques de carbone en graphite. Le tert-butyl (furan-2-yméthyl) (4-chloro-phenyl) carbamate (719 mg ; 2,34 mmol), l'électrolyte support : le tétrafluoroborate de tétraéthyle ammonium (75 mg ; 0,37 mmol) et le médiateur rédox : le bromure d'ammonium (295 mg, 3,04 mmol) sont introduits dans la cellule et dissous dans 25 mL de méthanol. Les électrodes sont ensuite insérées dans la cellule et branchées à un générateur, l'intensité de courant imposée est fixée à 20 mA. Le milieu réactionnel est homogénéisé par agitation magnétique. L'électrolyse est suivie par analyse CPG en effectuant des prélèvements du milieu réactionnel et elle est arrêtée lorsque le tert-butyl (furan-2-yméthyl) (4-chloro-phenyl) carbamate est totalement consommé. Une fois l'électrolyse terminée, le contenu de la cellule est transvasé dans un ballon. Après évaporation du solvant, le brut réactionnel est repris dans du diéthyl éther : l'électrolyte support précipite. Après filtration, la phase éthérée est évaporée et l'huile obtenue est traitée en milieu acide par un mélange THF/HCl (5N). Après réaction, le chlorure de 1-(4-chlorophenyl)-3-hydroxypyridinium est obtenu par évaporation du mélange réactionnel, précipitation dans l'acétate d'éthyle et filtration (226 mg, 40%). 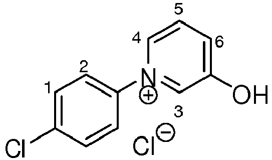
   **Aspect** : poudre blanche, **Tf :** 176,2 °C
   **IR:** 3375 (O-H), 3132 (=CH), 3088 (=CH), 3052(=CH), 1560 (C=C), 1470(C=C), 1386 (O-H), 1288 (O-H), 1258 (C-O), 1212 (C-O), 894 (C-H), 833 (C-H), 795 (C-H) cm⁻¹.
   **¹H NMR** (400 MHz, DMSO-d6): δ=8.80 (s, 1H, H-**3**), 8.76 (d, *J₄.₅*=*8 Hz*, 1H, H-**4**), 8.17 (d, *J₆₋₅*=*9* Hz, 1H, H-**6**), 8.08 (dd, *J_{5.4}*=*8 Hz*, *J₅₋₆*=*9Hz,* 1H, H-**5**), 7.89 (d, *J₁₋₂*=*8 Hz*, 2H, H-**1**), 7.83 (d, *J₂₋₁*=*8 H_{z}*, 2H, H-**2**).
   **¹³C NMR** (100 MHz, DMSO-d6): δ=157.1, 141.5, 135.9, 135.8, 133.2, 132.4, 130.0, 128.5, 126.6.
   **LC/MS m/z** (%) (colonne Betasil Phenyl-Hexyl 150 mm (150 mm x 2,1 mm x 3 µm), élution : MeOH/solution aqueuse de tampon formique (50/50), mode isocratique, D=0,2 mL/min, Rt=2.29 min, mode ESI+) calculée pour C₁₁H₉ClNO⁺ [M]⁺ : 206,04 (100,0%), 208,03 (32,0%), 207,04 (12,0%), 209,04 (3,9%) ; trouvée 206.15 (100.0%), 208.16 (29.9%), 207.20 (11.6%), 209.20 (3.7%).
   **Analyse élémentaire** calculée pour C₁₁H₉Cl₂NO. 5/4 H₂O : C (49.93 %), H (4.38 %), N (5.29 %); trouvée : C (50.18 %), H (4.08 %), N (5.82 %)

### • Préparation d'un analogue de formule I dans lequel R₄ est H: le Chlorure de 1-(4-chlorophenyl) pyridinium.

***(i)*** ***Préparation du sel de Zincke** :* Dans un schlenk, sont introduits 2,00 g (9,90 mmol, 1 éq) de 1-chloro-2,4-dinitrobenzene et 0,80 mL (9,90 mmol, 1 éq) de pyridine dans 50 mL d'acétone. Le mélange est laissé sous agitation et chauffé à 60°C pendant deux jours. Au départ la solution est de couleur rouge, puis elle passe à une teinte orangée : le chlorure de 1-(2, 4-dinitrophenyl) pyridinium précipite, est récupéré par filtration sur fritté et lavé au diethyl éther (2,42 g, 87%).
***(ii)*** ***Réaction de transamination** :* Dans un schlenk, sont introduits 400 mg (1,42 mmol, 1 éq) de chlorure de 1-(2, 4-dinitrophenyl) pyridinium et 1,1 g (8,54 mmol, 6 éq) de 4-chloroaniline dans 15 mL d'éthanol. Le mélange est laissé sous agitation et chauffé à 85°C pendant dix jours. Après évaporation du solvant, le chlorure de 1-(4-chlorophenyl) pyridinium précipite dans l'acétate d'éthyle et est récupéré par filtration sur fritté (225 mg, 70%). 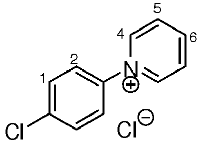
   **Aspect** : poudre marron clair, **Tf :** 123,4 °C
   **IR:** 3363 (O-H), 3115 (=CH), 3093 (=CH), 3020 (=CH), 1590 (C=C), 1474 (C=C), 1409 (O-H), 1361 (O-H), 1304 (O-H), 1261 (C-O), 1218 (C-O), 870 (C-H), 843 (C-H), 777 (C-H) cm⁻¹.
   **¹H NMR** (400 MHz, DMSO-d6): δ=9.34 (d, *J_{3.4}*=*8 Hz,* 2H, H-**3**), 8.80 (t, *J_{5.4}*=8 Hz, 1H, H-**5**), 8.32 (t, *J₄₋₃*=*J₄₋₅*=*8Hz*, 2H, H-**4**), 7.94 (d, *J₁₋₂*=*8 Hz*, 2H, H-**1**), 7.86 (d, *J₂₋₁*=*8 Hz*, 2H, H-**2**) ppm.
   **¹³C NMR** (100 MHz, DMSO-d6): δ=146.8, 145.0, 141.5, 136.0, 130.0, 128.0, 126.8.
   **LC/MS m/z** (%) (colonne Betasil Phenyl-Hexyl 150 mm (150 mm x 2,1 mm x 3 µm), élution : MeOH/solution aqueuse de tampon formique (50/50), mode isocratique, D=0,2 mL/min, Rt=2.29 min, mode ESI+) calculée pour C₁₁H₉ClN⁺ [M]⁺ : 190.04 (100.0%), 192.04 (32.0%), 191.05 (12.0%), 193.04 (3.9%) ; trouvée 190.18 (100.0%), 192.21 (30.8%), 191.23 (12.3%), 193.20 (3.2%).
   **Analyse élémentaire** calculée pour C₁₁H₉Cl₂N. H₂O : C (54.12 %), H (4.54 %), N (5.74 %); trouvée : C (54.55 %), H (4.58 %), N (5.72 %).

### Partie B : Evaluations Biologiques

### • Matériel et méthodes

### 1- Culture des cellules

Les cellules de neuroblastomes humains SH-SY5Y ont été cultivées dans un milieu DMEM contenant 4.5g.L⁻¹ de glucose, 10 % de sérum de veau foetal et 1% de pénicilline/streptomycine, et placées dans un incubateur à 37 °C avec 7 % de CO₂.

Au cours des différentes expériences, les cellules SH-SY5Y ont été ensemencées à une densité de 4x10⁴ cellules/mL. 24h après l'ensemencement, les cellules ont été exposées aux différentes molécules à tester à 37 °C pendant 24 h, 48 h, 72h ou 96 h.

Les fibroblastes de peau ont été obtenus à partir d'une biopsie de peau du dos de jeunes rats Wistar (3 mois). Les biopsies ont été coupées tangentiellement à l'épiderme pour séparer des fibroblastes superficiels et profonds du derme. Les cellules ont été cultivées dans du DMEM contenant 4500 mg/L de Glucose, 20 % SVF et 100 IU/mL de pénicilline/streptomycine, à 37°C avec 5 % de CO2. Les cellules ont été utilisées au 3ème passage. (Yue et al, CDD, 2009)

### 2- Test de viabilité cellulaire au MTT

Ce test a consisté à mesurer rapidement l'activité métabolique cellulaire en présence de la molécule étudiée à différentes concentrations afin d'effectuer une étude relation dose - réponse. Pour cela un sel de tétrazolium, le MTT, 3-(4,5-diméthylthiazol-2-yl)-2,5-diphenyltetrazolium bromide (Amoroso et al., Biochim Biophys Acta. 1999, 1452(2):151-60) a été mis au contact des cellules. Le MTT est réduit en formazan par les cellules vivantes qui possèdent la capacité à métaboliser celui-ci, contrairement aux cellules mortes. Pour effectuer ce test, des cellules neuronales SH-SY5Y, dans des plaques 48 puits, ont été incubées pendant 24h, 48h, 72h et 96h avec le pyridinium du furosémide à des concentrations allant de 0 à 1000 µM. Puis du MTT (0,5 mg/ml final) a été ajouté dans chaque puits et incubé à 37°C pendant 2h. A la fin de l'incubation, après avoir éliminé délicatement le milieu, du DMSOa été ajouté (200µL) pour solubiliser les membranes cellulaires et les cristaux de formazan formés par les cellules vivantes. La toxicité de la molécule a été évaluée en déterminant la quantité de MTT réduit, par mesure de l'absorbance à 550 nm, celle-ci étant proportionnelle à la quantité de cellules en vie. Les résultats ont été exprimés en fonction des valeurs obtenues pour des cellules traitées uniquement avec le solvant utilisé pour solubiliser la molécule d'intérêt. Afin de comparer la toxicité du pyridinium de furosémide à celle du MPP+, les cellules ont été incubées en présence de MPP+ (0 à 1000 µM) pendant 6 h. Le milieu de culture a été renouvelé et les cellules ont été incubées à 37°C pendant 24 h supplémentaires (en l'absence de MPP+). La survie cellulaire est mesurée à l'issue de ces 24 h d'incubation comme décrit ci-avant. Lors de l'évaluation de l'effet de différentes concentrations de l'α,-tocophérol (vitamine E), un antioxydant membranaire, les cellules ont été pré-traitées pendant 24h par cet antioxydant avant d'être exposées au pyridinium du furosémide à la concentration de 0,5mM dans les mêmes conditions expérimentales que précédemment.

### 3- Activité Caspases-9, -3 et -8 (Yue et al, Cell Death Differ. 2009 16(5):770-81.)

La mesure des activités des différentes caspases est basée sur la capacité des caspases à cliver des substrats spécifiques fluorescents: Ac-DEVD-AFC pour la caspase-3, Ac-IETD-AFCpour la caspase-8, ou Ac-LEHD-AFC pour la caspase-9. Les cellules neuronales SH-SY5Y, dans des plaques 48 puits, ont été incubées, soit avec le pyridinium de furosémide (1 mM) pendant 96h, soit avec le MPP+ (1 mM) pendant 6h puis 24h d'incubation (sans MPP+). Après incubation, les cellules ont été, après traitement par la trypsine, rinçage au PBS et comptage, récoltées dans 100 µl de tampon contenant HEPES 30 mM, EDTA 0,3 mM, NaCl100 mM, Triton X-100 0.15% et DTT 10 mM. L'extrait cellulaire a été ensuite incubé 1 heure à 37 °C avec le substrat spécifique (100 µM) dans une plaque 96 puits. La fluorescence a été mesurée à λₑₓ400 nm et λₑₘ505 nm dans un lecteur de plaque à détection de fluorescence (TECAN M1000 infinité) (Yue et al, supra). Les résultats ont été exprimés en pourcentage par rapport à l'activité mesurée pour des cellules cultivées dans les mêmes conditions et n'ayant subi aucun traitement.

### 4- Accumulation cellulaire d'a-synucléine (Lehri-Boufala et al, soumis)

Les cellules ont été incubées dans les mêmes conditions que précédemment avec le pyridinium du furosémide (1 mM, incubation : 96h) et le MPP+ (1mM, incubation : 6 h attente 24h). A l'issue de l'incubation, les cellules ont été traitées par la trypsine, rinsées au PBS, comptées, puis récoltées dans 100 µl de tampon contenant 250mM sucrose; 70mM KCl; 137mM NaCl; 4.3mM NaH₂PO₄; 1,4mM KH₂PO₄, pH 7,2; 250µg/ml de digitonine. Une incubation à 4°C a été ensuite effectuée pendant 5 minutes avant de centrifuger 5 minutes à 13000g afin de séparer la fraction cytosolique et lysosomale de la fraction mitochondriale. Les dépôts de la fraction cytosolique dans le western blot ont été ajustés en fonction du nombre de cellules. Les échantillons protéiques ont été dilués dans du Laemli 4X et ont été ensuite chauffés 3 minutes à 95°C afin de les dénaturer. Ils ont été introduits dans les puits du gel. En plus des échantillons, un marqueur de poids moléculaire (Fermentas) a été déposé dans le gel. La migration a été effectuée dans du tampon de migration (25 mM Tris pH8, 192 mM Glycine, 2 % SDS -20 %) à température ambiante à 100V jusqu'à ce que le front bleu de migration ait atteint le bas du gel. Le transfert a été réalisé sur une membrane PVDF. Le transfert s'est effectué dans du tampon de transfert (25 mM Tris, 192 mM Glycine, 10 % méthanol) pendant une nuit à 4°C et à 30V ou pendant 2 heures, à 4°C et à 80V. La saturation de la membrane a été effectuée dans du PBS 1X / Tween 0,2 % / Lait 3% pendant 1 heure à température ambiante. Ensuite, les membranes ont été immuno-marquées pendant 1,5 h à température ambiante avec un anticorps primaire dilué dans du PBS 1X / Tween 0,2 % / Lait 3%. Après 3 lavages en PBS 1X / Tween 0,2 %, l'immunomarquage a été révélé après une incubation pendant 1 h à température ambiante avec un anticorps secondaire couplé HRP (*HorseRadish Peroxydase*)*.* L'utilisation d'un substrat Chemiluminescent P qui réagit avec la peroxydase a permis de révéler la présence de la protéine d'intérêt sur la membrane grâce au dépôt d'un film photo pendant un temps précis. L'anticorps primaire utilisé est l'anti-α-synucléine 1:500 (visualisé avec un anticorps secondaire anti-lapin 1:10 000).

### 5- Extraction des mitochondries de cerveau de Rat (Brain Res. 2001 May 4;900(1):72-9.)

Pour extraire les mitochondries, un rat a été anesthésié par du Rompun 2% (100µL/100g) en utilisant une dose d'anesthésiant qui ne modifie pas l'activité de la chaine respiratoire mitochondriale. L'animal a été ensuite décapité pour prélever au sein du cerveau soit le cortex, soit le striatum qui ont été placés rapidement dans un Potter Elvejhem® contenant 6 ml de tampon d'extraction à 4°C (Sucrose 250mM, KCl 40mM, EGTA 2mM, BSA 1mg/ml, Tris 20mM, pH=7.2). Ce mélange a été homogénéisé afin de casser les cellules et de libérer les mitochondries puis centrifugé à 2000g pendant 8 minutes pour éliminer dans le culot les gros débris membranaires et les noyaux. Le surnageant a été ensuite récupéré et centrifugé à 13000g pendant 11 minutes. Le surnageant a été éliminé tandis que le culot contenant une fraction enrichie en mitochondries a été resuspendu dans 500µl de tampon de respiration à 4°C (D-mannitol 300mM, KCl 10mM, KH₂PO₄ 10mM, MgCl₂ 5mM, pH=7,2). Les protéines totales ont été dosées par la technique BCA Biorad® afin d'ajuster/normaliser la quantité de protéines mitochondriales utilisées dans les expériences.

### 6- Mesure de la consommation d'oxygène et fonctionnement de la chaine respiratoire (Morin et al, Neurosciences,. 2002;115(2):415-24.)

Dans la cuve de polarographie (Hansatech), 30 µL de solution de mitochondries (0,2mg), 460µL de tampon de respiration, 5µL de la solution de la molécule à tester (c'est-à dire MPP+ ou pyridinium de furosémide) (ou de son solvant de solubilisation) ont été ajoutés. Puis 5 µl du mélange de substrats malate-pyruvate à 10/10mM ont été ajoutés, ce qui permet le fonctionnement de la chaîne respiratoire (stade 2) à partir du complexe I. Après 40 secondes environ, 5µL d'ADP à 200µM ont été ajoutés, entrainant une augmentation de la consommation d'oxygène car l'ADP active le fonctionnement du complexe V (Stade 3). Une fois tout l'ADP consommé, la consommation d'oxygène revient à un stade initial (Stade 4) équivalent au Stade 2. L'effet de la molécule a été évalué sur le contrôle respiratoire, (rapport des stades 3 sur 4) qui correspond au couplage de la chaine respiratoire, c'est-à-dire de la synthèse d'ATP au niveau du complexe V et de la consommation d'oxygène due aux complexe I à IV. Les résultats sont exprimés en pourcentage de la valeur mesurée pour des mitochondries non-traitées avec la molécule testée (mais traitées par le solvant de solubilisation).

Après avoir examiné le fonctionnement global de la chaine respiratoire, le complexe I a été bloqué par l'ajout de roténone afin de déterminer le complexe cible de la molécule. Le même protocole a été utilisé mais avec l'ajout de succinate à 10mM puis de roténone à 2µM. Le succinate a pour effet d'induire le fonctionnement de la chaine respiratoire par le complexe II, en favorisant le passage de FADH au niveau de ce complexe, tandis que la roténone inhibe le fonctionnement du complexe I.

### 7- Dosage de l'activité du complexe I par spectrophotométrie.

La cinétique de la réaction d'oxydation du NADH en NAD+ à 340 nm à 37°C, est suivie en continu par spectrophotométrie à l'aide d'un spectrophotomètre JASCO.V530 ou d'un lecteur de plaque TECAN infinite 1000M, paramétré à une longueur d'onde de 340 nm pendant 3 min. La quantité d'extrait de mitochondrie pour obtenir l'activité maximale introduit dans le volume réactionnel de 100µg avec le spectophotomètre et de 10µg pour le lecteur. Chaque manipulation doit être effectuée à saturation de substrat, soit une concentration en NADH de 160µM. Les mitochondries préalablement réparties en plusieurs aliquotes ont été congelées puis décongelées rapidement quelques minutes avant la manipulation. Dans une première étape, l'extrait de mitochondries est introduit dans le tampon hypotonique (50 mM de KH₂PO₄ et 10 mM de MgCl₂, pH=7,2 à 37°C) pendant 2 minutes de manière à éclater les membranes des mitochondries et accéder à la chaine respiratoire (Chretien et al.Biochem Biophys Res Commun. 2003,301(1):222-4). De l'albumine sérique bovine (3.5mg/ml), du décylubiquinone (10Mm), du KCN (10mM) et du NADH sont ajoutés avant de démarrer l'enregistrement de la cinétique. L'albumine sérique bovine permet d'optimiser l'activité du complexe I (Chretien et al., *supra* ; Janssen et al. Clin Chem, 2007, 53(4):729-34). Le délubiquinone remplace l'accepteur naturel des électrons du complexe I, le coenzyme Q₁₀ ou ubiquinone. Le cyanure est un inhibiteur du complexe IV de la chaine respiratoire. La pente de la cinétique est ensuite mesurée. Les résultats obtenus en présence de la molécule d'intérêt sont comparés aux témoins réalisés uniquement avec le solvant de la molécule. Le pourcentage d'inhibition est alors calculé.

### • Résultats

### 1- Evaluation de la viabilité cellulaire, test MTT

Après 24 h d'incubation, le pyridinium de furosémide n'a pas d'effet significatif sur la survie cellulaire, même à une concentration de 1 mM. A contrario, le MPP+ induit une mort cellulaire massive à une concentration 1 mM après une durée d'incubation de 6h des cellules (+ 24 h sans MPP+) (Figure 1A). L'EC50 du MPP+ est de 386,2 ±19.2 µM.

En revanche, après 96 h d'incubation avec le pyridinium de furosémide, la viabilité cellulaire a été abaissée d'environ 60% . L'EC₅₀ du pyridinium de furosémide est estimée à 0,973µM (voir Figure 1B).Le pyridinium du furosémide exerce donc un effet toxique sur les cellules neuronales SH-SY5Yobservables après une'une longue exposition.

Les Figures 1A et 1B illustrent clairement la différence de toxicité cellulaire existant entre le MPP+ et le pyridinium de furosémide.

### 2- Activation des Caspases

Les mesures d'activité caspase sont illustrées à la Figure 1C.

L'activité enzymatique de la caspase 9 a été significativement augmentée (170%) dans les cellules traitées avec du pyridinium de furosémide pendant 96 h en comparaison à l'activité de cette protéase mesurée dans les cellules non traitées. Cette augmentation est cependant significativement plus faible que celle observée après incubation de 24h avec le MPP+. Après incubation avec le pyridinium de furosémide, l'activité de la caspase 3 dans les cellules a été augmentée de manière significative (179%) comparée à l'activité mesurée dans les cellules non traitées. Cette augmentation est du même ordre que celle observée après une incubation avec le MPP+ de seulement 24h avec le MPP⁺. Les cellules après incubation avec le pyridinium de furosémide présentent des activités cytosoliques des caspases 9 et 3 significativement plusélevées que les cellules non traitées. Ceci indique que c'est la voie intrinsèque mitochondriale de l'apoptose qui est certainement activée pour diminuer la survie cellulaire en induisant la mort des cellules, comme observé pour le MPP+. Toutefois, la cinétique pour obtenir cet effet est beaucoup plus lente (96h) pour le pyridinium du furosémide que pour le MPP+ qui ne nécessite que 6h (+ 24 h sans incubation de MPP+). L'exposition des cellules au pyridinium de furosémide (96 h) ou au MPP+ (6h), n'induit pas de modifications significatives de l'activité de la caspase 8. Ces molécules n'induisent pas la voie extrinsèque de l'apoptose.

### 3- Accumulation cellulaire d'a-synucléine

L'évaluation de l'accumulation d'α,-synucléine a été effectuée dans les cellules SH-SY5Y, après 96h d'incubation en présence du pyridinium de furosémide ou du DMSO(solvant de solubilisation - témoin). En parallèle, l'accumulation d'α,-synucléine a été déterminée pour des cellules SH-SY5Y après 6 h d'incubation avec le MPP+ (+ 24h sans MPP+) ou le DMSO (témoin). Après traitement des cellules, la quantité totale d'α,-synucléine a été déterminée par une technique d'immunotransfert (western blot) permettant la séparation et l'identification de la protéine d'intérêt, l'α-synucléine, par un anticorps spécifique. La Figure 2A présente les gels obtenus après révélation et la Figure 2B le résultat de quantification de l'accumulation totale d'a-synucléine (sous forme monomère, dimère et oligomère). Une augmentation significative de la quantité d'α,-synucléine totale de 40 % a été constatée dans les cellules traitées au pyridinium de furosémide par rapport aux cellules témoins. Cette augmentation est d'un point de vue quantitatif beaucoup moins importante que celle induite par le MPP+ (319%), et ce malgré un temps d'incubation beaucoup plus long. Ceci confirme que le pyridium de furosémide a une cinétique de toxicité lente *in vitro,* avec une accumulation d'α-synucléine impliquant des processus progressifs et lents, probablement très différents de ceux, « brutaux », induits par le MPP⁺.

### 4- Effets sur le fonctionnement de la chaine respiratoire des mitochondries de cortex cérébral de Rat

Les courbes d'effet dose-réponse sur le contrôle respiratoire sont présentées à la figure 3A (MPP+ : ronds blancs ; pyridinium de furosémide : carrés noirs). En augmentant la concentration de MPP+ ou de pyridinium de furosémide, le contrôle respiratoire des mitochondries de cortex cérébral induit par du malate/pyruvate a été diminué. La consommation d'oxygène diminue en présence de ces deux molécules, suggérant qu'elles possèdent une cible au niveau de la chaine respiratoire.

### 5- Effets sur le fonctionnement de la chaine respiratoire des mitochondries de striatum de Rat

Les courbes d'effet dose-réponse sur le contrôle respiratoire sont présentées à la figure 3B (MPP+ : ronds blancs ; pyridinium de furosémide : carrés noirs)

Au niveau du striatum, nous avons également observé une diminution de la consommation d'oxygène. Notablement, l'effet du pyridinium de furosémide a été plus important sur les mitochondries de striatum que sur celles extraites de cortex. Ceci suggère qu'il y existe une spécificité du pyridinium du furosémide vis-à-vis du striatum.

### 6- Effets sur le fonctionnement des complexes II à V de la chaine respiratoire des mitochondries de cortex cérébral de Rat

En induisant le fonctionnement de la chaîne respiratoire par le complexe II (après inhibition du Complexe I par la roténone), le contrôle respiratoire des mitochondries de cortex n'a pas été modifié même en présence d'une concentration élevée (1 mM) de MPP⁺ ou de pyridinium du furosémide (Figure 4A). La chaine respiratoire fonctionnait normalement. Ces expériences ont donc montré que le furosémide de pyridium, tout comme le MPP+, altère le fonctionnement mitochondrial par inhibition du complexe I.

### 7- Effet protecteur de différentes molécules vis-à-vis de la toxicité induite par le pyridinium du furosémide

Un prétraitement des cellules SH-SY5Y par l'α,-tocophérol aux concentrations de 0,01 et 0,1 µg/mL, (sans incubation subséquente avec le pyridinium furosémide) n'a pasmodifié la survie cellulaire. Une légère augmentation de 20% a toutefois été observée pour 1 µg /mL. L'incubation avec le pyridinium de furosémide à 0,5mM a induit une diminution de la survie cellulaire de l'ordre de 20%. Un prétraitement de 24h par l'a-tocophérol avant l'incubation avec le pyridinium de furosémide a permis d'augmenter le taux de survie cellulaire de manière dose dépendante, avec un effet visible dès 0,1 µg/mL d'α-tocophérol. Ceci suggère que la toxicité du pyridinium du furosémide sur la survie cellulaire peut être contrée, et qu'elle fait intervenir la production de radicaux libres de l'oxygène puisque le traitement par un antioxydant tel que l'α,-tocophérol (anti-oxydant) est capable de protéger les cellules.

### 8- Evaluation de l'effet d'analogues du pyridinium de furosémide sur l'activité du complexe I in vitro.

La capacité de différents analogues du pyridinium de furosémide à inhiber le complexe mitochondrial I a été évaluée comme décrit au point 7 de la partie « matériel et méthode » ci-avant. Ces tests d'activité ont mis en évidence que le 1-(4-chlorophenyl)-3-hydroxypyridinium induit une inhibition complète de l'activité du complexe I à la concentration de 0,2mM. Il a été également montré que les analogues pour lesquels R₄ = H sont également capables d'inhiber, au moins partiellement, le complexe I. La capacité d'inhibition de ces analogues varie en fonction de la nature des substituants R₁, R₂ et R₃, ce qui suggère l'existence d'une relation structure-activité précise. Ainsi, le composé non-hydroxylé le 1-(4-chlorophenyl) pyridinium a inhibé de manière quasi totale (95% d'inhibition) l'activité du complexe I. La substitution du chlore par un méthyl en R₂, a diminué l'effet inhibiteurmais le complexe I reste néanmoins inhibé de 52%.

### Partie C : étude du modèle chimique et test de criblage

### 1 - Evaluation in vivo du pyridinium de furosémide chez le rat

### • Matériel et Méthode

Des rat albinos mâles Sprague Dawley (adulte pesant 900-1000g) sont hébergés dans une pièce à température contrôlée avec un accès libre à la nourriture et à l'eau.

Une plateforme de monitoring électrophysiologique et comportementale du rat est utilisée. Pour cela, un implant (Data Science) a été installé en sous cutané chez un rat et a permis l'enregistrement de l'électroencéphalogramme par télémétrie, en laissant l'animal totalement libre de ses mouvements. La cage a été équipée d'une caméra vidéo (10 images par secondes) et d'un éclairage infrarouge permettant l'enregistrement de l'activité de l'animal. L'EEG et la vidéo ont été enregistrés en séquences de 10 secondes, sur chacune desquelles un ensemble de variable est calculé. L'utilisation d'un logiciel spécifique permet en intégrant ces variables sur l'ensemble de l'enregistrement de détecter des changements dans l'activité de l'animal, son EEG, ses rythmes circadiens. Chaque animal est son propre témoin car il a été enregistré pendant 5 jours, avant l'administration du le pyridinium de furosémide. La solution de pyridinium de furosémide (20 mg) a été solubilisée dans du diméthylsulfoxyde (DMSO, 200µl) puis diluée dans l'eau (50 ml). La solution a été renouvelée tous les jours et administrée à raison de 20 mg/kg dans l'eau de boisson pendant 7 jours. Les rats ont été ensuite sacrifiés 5 semaines après la dernière administration. Les rats ont été anesthésiés avec du pentobarbital et perfusés par le coeur avec une solution saline (NaCl 9%) et fixés avec du paraformaldéhyde 4%. Après la perfusion, le cerveau a été retiré et placé dans NaCl (0,9%) à 4 ° C. Le cerveau a été congelé dans du Tissue Tek immergé dans un bain d'isopentane froid. Des sections coronales de huit micromètres ont été effectuées à l'aide d'un cryostat et ont été traitées pour immunocytochimie (marquage de l'alpha-synucléine).

### • Résultats

Comme cela est montré par les figures 5A et 5B qui présentent les résultats d'immunomarquage de coupes coronales, l'exposition au pyridinium du furosémide par voie orale, a induit une augmentation nette de l'alpha-synucléine dans le striatum des animaux. Les premiers résultats de l'étude comportementale ont montré, par ailleurs, une diminution importante de l'amplitude de l'électroencéphalogramme, 5 semaines après la fin du traitement, comparé à l'amplitude avant exposition au pyridinium de furosémide (Figure 6A). Les résultats obtenus sur les phases d'éveil, et de sommeil n'indiquent pas de modifications de ces paramètres par l'exposition au pyridinium de furosémide. Seul, le nombre de phases de sommeil paradoxal est nettement augmenté (x2), 5 semaines après la fin de l'exposition au pyridinium (Figure 6D). Cette augmentation de phases de sommeil paradoxal est fréquemment observée chez les malades atteints de maladie de Parkinson et dans les synucléinopathies. Dans les modèles animaux, l'obtention de tels phénomènes n'a pas été observée, à ce jour, chez la souris, après traitement au MPTP (Laloux et al. Exp Brain Res. 2008 186(4):635-42).

### 2- Evaluation in vivo du pyridinium de furosémide chez la souris

### • Matériel et méthodes

Des Souris Swiss ont été réparties en deux groupes de 5 animaux (expérience et témoin). Les expériences ont été réalisées 2 fois de manière indépendante. Les souris du groupe expérience ont été exposées au pyridinium du furosémide pendant 7 jours, à raison d'une dose journalière de 20 mg/kg. Le pyrimidium de furosémide a été administré par voie orale, dans l'eau de boisson. Les souris ont été sacrifiées 30 jours après la fin du traitement au pyridinium de furosémide.

*Immunohistochimie :* A cette fin, les souris ont été anesthésiées avec du pentobarbital (50 mg/Kg), une perfusion intra-cardiaque de NaCl (0.9%) est alors réalisée et suivie d'une fixation par perfusion de paraformaldéhyde à 4% dans du tampon phosphate 0.1 M, pH 7,2. Le cerveau a été retiré, placé dans NaCl (0.9%) à 4 ° C puis congelé dans du Tissue Tek immergé dans un bain d'isopentane froid. Des sections coronales de huit micromètres ont été effectuées à l'aide d'un cryostat et ont été traitées par immunohistochimie (marquage de la tyrosine hydroxylase et de l'alpha-synucléine phosphorylée en Ser129).

*Activité mitochondriale :* Pour chaque souris sacrifiée, des échantillons de cortex et de stratium ont été préalablement prélevés et traités pour extraire les mitochondries. L'activité de la chaîne respiratoire mitochondriale et celle du complexe I ont été déterminées comme décrit dans la partie B ci-avant, dans la sous-partie « matériel et méthode » (sans ajout dans le milieu de pyridinium de furosémide ou d'analogue de celui-ci).

### • Résultats

1- Concernant l'activité mitochondriale, on a observé une diminution de 28% du contrôle respiratoire et une diminution de 49% de l'activité du complexe I au niveau de la chaîne respiratoire des mitochondries provenant du striatum des souris exposées au furosémide de pyridinium, par rapport aux activités mesurées au niveau du stratium des animaux contrôles (Figure 7).
   L'exposition au pyridinium du furosémide est donc capable d'induire une dysfonction des mitochondries du striatum, en ciblant le complexe I de la chaine respiratoire. Cette inhibition de la chaîne respiratoire est toujours effective 30 jours après la fin de l'exposition au pyridinium de furosémide.
**2-** Concernant les études d'immunohistochimie, on a observé une nette diminution du marquage de la tyrosine hydroxylase au niveau du striatum des souris exposées au pyridinium du furosémide (Figure 8). Une augmentation très nette de l'a-synucléine phosphorylée sur la sérine en position 129 a aussi été mise en évidence dans le striatum des souris exposées au furosémide de pyridinium, par immunomarquage avec un anticorps spécifique de l'α-synucléine phosphorylée à cette position (Figure 9). Cette augmentation de l'a-synucléine phosphorylée est spécifique du stratium puisqu'aucune augmentation n'a été observée dans le cortex des souris exposées au furosémide de pyridinium.

Des études antérieures ont montré que l'α-synucléine phosphorylée en Ser129 représente -90% de l'alpha-synucléine contenue dans les corps de Lewy, alors qu'elle ne représente que 4% de l'alpha-synucléine présente dans les tissus cérébraux sains (Fujiwara, H et al, Nat. Cell Biol.2002, 4, 160-164) ; Anderson, J. P et al, J. Biol. Chem., 2006, 281, 29739-29752). De manière notable, l'augmentation de l'a-synucléine phosphorylée en Ser-129 n'a jamais été observée, à ce jour, dans les modèles chimiques de la maladie de Parkinson décrits dans l'état de la technique (modèle induit par le MPTP, ou par le roténone par exemple).

### 3- Exemple de mise en oeuvre d'un test de criblage selon l'invention

Les embryons de poisson zèbre sont élevés dans le milieu E3 (5 mM de NaCl, 0,17 mM KCl, 0,33 mM de CaCl₂, 0,33 mM MgSO4, pH 7,4) à 28,5 ° C avec un cycle jour-nuit 14:10 h. Le milieu est remplacé tous les jours jusqu'au 3 dpf (day post fecondation). Après 6 dpf, les larves sont nourries deux fois par jour. Pour l'analyse des mouvements, les larves sont soigneusement transférés dans les puits d'une plaque 48 puits remplis de milieu E3 à l'aide d'une pipette Pasteur de gros calibre (au moins 4 mm) pour éviter tout dommage mécanique de la larve. Celles-ci sont laissées pendant 30 min à 28,5 °C dans la plaque avant l'enregistrement afin qu'elles s'acclimatent à l'environnement des puits. Le traitement par le pyridinium de furosémide est effectué par une exposition à différentes concentrations de pyridinium diluées dans du milieu E3 à partir de 48h après dpf des larves de poisson zèbre. Les concentrations testées iront de concentrations proches de celles retrouvées dans l'environnement pour le furosémide (0,200 ng/L) à des concentrations plus pharmacologiques (1 mg/L). Dans le but d'évaluer le potentiel protecteur de nouvelles molécules pharmacologiques, un prétraitement par un anti-oxydant par exemple comme l'alpha-tocophérol, sera effectué pendant les 24h précédant l'exposition au pyridinium du furosémide. Le test de criblage sera ensuite constitué par l'association de différentes méthodologies et approches qui permettront de progressivement sélectionner les molécules protégeant des effets du pyridinium du furosémide, puis de comprendre les mécanismes biologiques soutenant ces effets protecteurs.

Evaluation comportementale : La première étape du criblage sera effectuée en mesurant les effets comportementaux des molécules d'intérêt sur les larves de poisson zèbre. Les capacités de nage, les déplacements des larves de poisson zèbre exposées au pyridinium du furosémide seront évalués en présence et en absence des molécules d'intérêt. Les capacités de nage, les déplacements des larves seront évalué en utilisant un appareil de type ZebraBox®. Au cours de toute la durée de l'étude, les mêmes larves seront évaluées, permettant ainsi de réduire le nombre d'animaux utilisés conformément à la règle des « 3R » régissant les expérimentations animales.

Evaluation par immunohistologie : Ce criblage peut aussi être mené en s'intéressant à la présence d'agrégats d'alpha-synucleine, de corps de Lewy ainsi qu'à la dégénérescence des neurones dopaminergiques, tant par leur nombre que par l'expression de la tyrosine hydroxylase. Ces études seront réalisées en fixant puis perméabilisant les larves après traitement au jour voulu et en révélant la présence de la protéine d'intérêt à l'aide d'anticorps spécifiques par immunohistologie. Le résultat pourra être alors observée au microscope ou quantifiée dans un lecteur de plaque à fluorescence et ne nécessite qu'une journée pour être réalisée.

Evaluation biochimique : Ces études sont réalisées sur des broyats ou des extraits protéiques des larves de poisson zèbre. Ainsi une évaluation du fonctionnement de la chaine respiratoire mitochondriale, de l'activité du complexe I, du stress oxydatif, de l'apoptose pourront être effectuées. Ceci permettra de caractériser le/les mécanismes d'action moléculaire de la molécule évaluée et potentiellement protégeant le tissus cérébral de la formation des marqueurs biologiques clef de la maladie de Parkinson et d'autres synucléinopathies.

Toutes les expériences sont effectuées en triple, avec cinq à dix résultats biologiques indépendants analysés sur des batteries de poisson zèbre provenant de différentes pontes.

## Revendications

1. Utilisation d'un composé de formule (I) 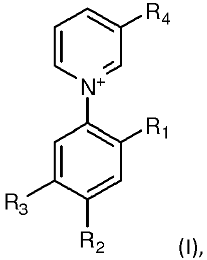 dans laquelle,
- R₁ représente un atome d'hydrogène, un alkyle en C₁-C₄, un atome d'halogène, -OH, -CN, -CF₃, -C(=O)NH₂, -C(=O)O⁻, ou -C(=O)O-R₅ avec R₅ représente H ou un alkyle en C₁-C₄,
- R₂ est -SO₂NH₂, un atome d'hydrogène, un atome d'halogène, un alkyle en C₁-C₄, -OH, -CN, -C(=O)NH₂, -CF₃, ou -C(=O)OR₆ avec R₆ représente H ou un alkyle en C₁-₄
- R₃ est H, un atome d'halogène, un alkyle en C₁-C₄,-OH, -CN,-C(=O)NH₂,-CF₃, ou - C(=O)OR₇ avec R₇ représente H ou un alkyle en C₁-C₄, et
- R₄ représente un atome d'hydrogène, -OH ou -OCH₃,
ou un sel acceptable sur le plan pharmaceutique de celui-ci,
dans la préparation d'un modèle chimique d'une synucléinopathie, de préférence de la maladie de Parkinson, par mise en contact d'un système cellulaire choisi parmi une cellule, une culture cellulaire, un tissu et un animal non-humain avec ledit composé.

2. Méthode de préparation d'un modèle chimique d'une synucléinopathie, de préférence de la maladie de Parkinson, comprenant une étape de mise en contact d'un système cellulaire choisi parmi une cellule, une culture cellulaire, un tissu et un animal non-humain avec un composé de formule (I) tel que défini à la revendication 1 ou un sel acceptable de celui-ci.

3. Utilisation selon la revendication 1 ou méthode selon la revendication 2 dans laquelle le modèle chimique est obtenu à partir d'un tissu nerveux, de préférence cérébral, d'une culture organotypique obtenue à partir d'un tissu cérébral, d'une culture de cellules nerveuses, par exemple une culture de neurones dopaminergiques, d'une culture primaire de neurone ou d'une lignée cellulaire, de préférence d'origine neuronale.

4. Utilisation selon la revendication 3 ou méthode selon la revendication 3 dans laquelle le modèle chimique est obtenu à partir d'une lignée cellulaire choisie parmi le groupe constitué par les lignées SH-SY5Y, SK-N-MC et PC12.

5. Utilisation selon la revendication 1 ou méthode selon la revendication 2 dans laquelle le modèle chimique est un animal non-humain, de préférence choisi dans le groupe constitué par un mammifère non-humain, un invertébré tel que *C. elegans* ou un poisson tel que le poisson zèbre.

6. Utilisation selon la revendication 1, 3, 4 ou 5 ou méthode selon la revendication 2, 3, 4 ou 5 dans laquelle le composé de formule (I) est le pyridinium de furosémide.

7. Modèle chimique d'une synucléinopathie obtenu selon une méthode telle que définie dans l'une des revendications 2 à 6.

8. Utilisation d'un modèle chimique d'une synucléinopathie tel que défini à la revendication 7 pour la mise en oeuvre d'un test de criblage de composés destinés au traitement ou à la prévention de ladite synucléinopathie, de préférence de la maladie de Parkinson.

9. Utilisation d'un modèle chimique d'une synucléinopathie tel que défini à la revendication 7 pour évaluer l'efficacité d'un principe actif dans le traitement ou la prévention de ladite synucléinopathie, ledit modèle étant un modèle mammifère non-humain.

10. Procédé pour évaluer, cribler ou sélectionner un composé destiné au traitement ou à la prévention d'une synucléinopathie, de préférence la maladie de Parkinson, ledit procédé comprenant:
a) la mise en contact d'un système cellulaire choisi parmi une cellule, une culture cellulaire, un tissu et un animal non-humain avec un composé tel que défini à la revendication 1, de préférence le pyridinium de furosémide,
b) la mise en contact dudit système cellulaire avec le composé à tester, et
c) la détection ou la quantification d'un marqueur caractéristique de la synucléinopathie en vue de déterminer l'efficacité du composé à tester,
l'étape b) pouvant être mise en oeuvre simultanément, avant ou après l'étape a) ou chevaucher l'étape a).

11. Procédé selon la revendication 10 dans lequel :
- ledit système cellulaire est choisi parmi le groupe constitué par un tissu nerveux, de préférence cérébral, une culture organotypique obtenue à partir d'un tissu cérébral, une culture de cellules nerveuses, par exemple de neurones de préférence dopaminergiques et/ou de cellules gliales, et une lignée cellulaire de préférence d'origine neuronale, et
- ledit marqueur est choisi parmi le groupe constitué par une accumulation intracellulaire d'une synucléine, de préférence d'a-synucléine, un marqueur de la mort cellulaire par apoptose, de préférence une augmentation d'une activité caspase, une diminution du taux de survie cellulaire, une diminution de l'activité de la chaîne respiratoire mitochondriale et leurs combinaisons.

12. Procédé selon la revendication 10 dans lequel :
- ledit système cellulaire est un animal non-humain et
- ledit marqueur caractéristique de la synucléinopathie est choisi parmi le groupe constitué par un trouble moteur, en particulier un trouble de la mobilité ou une diminution de la vitesse de déplacement, une dégénérescence des neurones dopaminergiques, l'accumulation intracellulaire d'alpha-synucléine au niveau d'un neurone dopaminergique et leurs combinaisons.

13. Kit pour la mise en oeuvre d'un procédé pour évaluer, cribler ou sélectionner un composé destiné au traitement ou à la prévention d'une synucléinopathie, tel que défini dans la revendication 10, ou pour la préparation d'un modèle chimique tel que défini à la revendication 7 comprenant:
- un composé tel que défini à la revendication 1, de préférence le pyridinium de furosémide, et
- un système cellulaire choisi parmi une cellule, une culture cellulaire, un tissu et un animal non-humain destiné à être mis en contact avec le composé pour obtenir ledit modèle chimique.

14. Procédé selon la revendication 10 ou 12 ou kit selon la revendication 13 dans lequel le système cellulaire est un nématode tel que *C. elegans,* un poisson zèbre ou une larve de ceux-ci.

15. Utilisation d'un composé tel que défini dans la revendication 1, de préférence le pyridinium de furosémide, pour induire chez un mammifère non-humain un ou plusieurs symptômes associés à la maladie de Parkinson, de préférence choisi parmi le groupe constitué par une rigidité musculaire, un tremblement au repos, une instabilité posturale, une akinésie, un trouble du sommeil ou de l'éveil, une accumulation intracellulaire d'alpha-synucléine, de préférence sous la forme de corps de Lewy, une diminution de l'activité mitochondriale, en particulier du complexe I, et une dégénérescence des neurones dopaminergiques de la *substantia nigra*.

16. Utilisation d'un composé tel que défini dans la revendication 1, de préférence le pyridinium de furosémide, en tant que composé témoin ou en tant que composé étalon, dans un test d'évaluation de la neurotoxicité d'un composé, ledit test d'évaluation comprenant l'incubation d'un système cellulaire choisi parmi une cellule, une culture cellulaire, un tissu et un animal non-humain avec le composé pendant une durée supérieure à 48h.

## Patentansprüche

1. Verwendung einer Verbindung der Formel (I) 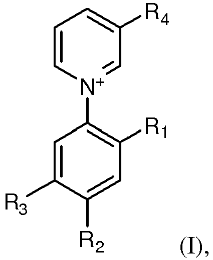 in der
- R₁ für ein Wasserstoffatom, ein C₁-C₄-Alkyl, ein Halogenatom, -OH, -CN, -CF₃, - C(=O)NH₂, -C(=O)O⁻ oder -C(=O)O-R₅ steht, wobei R₅ für H oder ein C₁-C₄-Alkyl steht,
- R₂ -SO₂NH₂, ein Wasserstoffatom, ein Halogenatom, ein C₁-C₄-Alkyl, -OH, -CN, - C(=O)NH₂, -CF₃ oder -C(=O)OR₆ ist, wobei R₆ für H oder ein C₁₋₄-Alkyl steht,
- R₃ H, ein Halogenatom, ein C₁-C₄-Alkyl, -OH, -CN, -C(=O)NH₂, -CF₃ oder -C(=O)OR₇ ist, wobei R₇ für H oder ein C₁-C₄-Alkyl steht, und
- R₄ für ein Wasserstoffatom, -OH oder -OCH₃ steht,
oder ein pharmazeutisch verträgliches Salz davon,
in der Herstellung eines chemischen Modells einer Synukleinopathie, vorzugsweise des Morbus Parkinson, mittels Inkontaktbringen eines Zellsystems, ausgewählt aus einer Zelle, einer Zellkultur, einem Gewebe und einem nichthumanen Tier, mit besagter Verbindung.

2. Verfahren zur Herstellung eines chemischen Modells einer Synukleinopathie, vorzugsweise des Morbus Parkinson, umfassend einen Schritt des Inkontaktbringens eines Zellsystems, ausgewählt aus einer Zelle, einer Zellkultur, einem Gewebe und einem nichthumanen Tier, mit besagter Verbindung der Formel (I), wie in Anspruch 1 definiert, oder einem verträglichen Salz davon.

3. Verwendung gemäß Anspruch 1 oder Verfahren gemäß Anspruch 2, wobei das chemische Modell von einem Nervengewebe, vorzugsweise Gehirngewebe, einer organotypischen Kultur, erhalten von einem Gehirngewebe, einer Nervenzellkultur, zum Beispiel einer Kultur dopaminerger Neuronen, einer primären Neuronenkultur oder einer Zelllinie, vorzugsweise neuronalen Ursprungs, erhalten wird.

4. Verwendung gemäß Anspruch 3 oder Verfahren gemäß Anspruch 3, wobei das chemische Modell von einer Zelllinie erhalten wird, ausgewählt aus der Gruppe, bestehend aus den SH-SY5Y-, SK-N-MC- und PC12-Zelllinien.

5. Verwendung gemäß Anspruch 1 oder Verfahren gemäß Anspruch 2, wobei das chemische Modell ein nichthumanes Tier ist, vorzugsweise ausgewählt aus der Gruppe, bestehend aus einem nichthumanen Säuger, einem Invertebraten wie *C. elegans* oder einem Fisch wie dem Zebrafisch.

6. Verwendung gemäß Anspruch 1, 3, 4 oder 5 oder Verfahren gemäß Anspruch 2, 3, 4 oder 5, wobei die Verbindung der Formel (I) Pyridinium-Furosemid ist.

7. Chemisches Modell einer Synukleinopathie, erhalten gemäß einem Verfahren, wie in einem der Ansprüche 2 bis 6 definiert.

8. Verwendung eines chemischen Modells einer Synukleinopathie, wie in Anspruch 7 definiert, für die Durchführung eines Durchmusterungstests von Verbindungen, die zur Behandlung oder Prävention besagter Synukleinopathie, vorzugsweise Morbus Parkinson, bestimmt sind.

9. Verwendung eines chemischen Modells einer Synukleinopathie, wie in Anspruch 7 definiert, für die Evaluierung der Wirksamkeit eines Wirkstoffes in der Behandlung oder Prävention besagter Synukleinopathie, wobei besagtes Modell ein nichthumanes Säugermodell ist.

10. Verfahren zur Evaluierung, Durchmusterung oder Auswahl einer Verbindung für die Behandlung oder Prävention einer Synukleinopathie, vorzugsweise Morbus Parkinson, wobei besagtes Verfahren umfasst:
a) Inkontaktbringen eines Zellsystems, ausgewählt aus einer Zelle, einer Zellkultur, einem Gewebe und einem nichthumanen Tier, mit einer Verbindung, wie in Anspruch 1 definiert, vorzugsweise Pyridinium-Furosemid,
b) Inkontaktbringen besagten Zellsystems mit der zu testenden Verbindung, und
c) Nachweis oder quantitative Bestimmung eines für die Synukleinopathie charakteristischen Markers zwecks Bestimmung der Wirksamkeit der zu testenden Verbindung,
wobei Schritt b) gleichzeitig, vor oder nach Schritt a) durchgeführt werden kann oder sich mit Schritt a) überschneiden kann.

11. Verfahren gemäß Anspruch 10, wobei:
- besagtes Zellsystem aus der Gruppe ausgewählt ist, bestehend aus einem Nervengewebe, vorzugsweise Gehirngewebe, einer organotypischen Kultur, erhalten von Gehirngewebe, einer Nervenzellkultur, zum Beispiel Neuronen, vorzugsweise dopaminerger Neuronen, und/oder Gliazellen, und einer Zelllinie, vorzugsweise neuronalen Ursprungs, und
- besagter Marker aus der Gruppe ausgewählt ist, bestehend aus einer intrazellulären Anhäufung eines Synukleins, vorzugsweise α-Synuklein, einem Marker des Zelltods durch Apoptose, vorzugsweise einer Erhöhung einer Caspase-Aktivität, einer Verringerung der Zellüberlebensrate, einer Verringerung der Aktivität der mitochondrialen Atmungskette und Kombinationen davon.

12. Verfahren gemäß Anspruch 10, wobei
- besagtes Zellsystem ein nichthumanes Tier ist, und
- besagter für die Synukleinopathie charakteristische Marker aus der Gruppe ausgewählt ist, bestehend aus einer motorischen Störung, insbesondere einer Störung der Mobilität oder einer Verlangsamung der Bewegungsgeschwindigkeit, einer Degeneration der dopaminergen Neuronen, der intrazellulären Anhäufung von alpha-Synuklein auf Ebene eines dopaminergen Neurons und Kombinationen davon.

13. Kit für die Durchführung eines Verfahrens zur Evaluierung, Durchmusterung oder Auswahl einer Verbindung für die Behandlung oder Prävention einer Synukleinopathie, wie in Anspruch 10 definiert, oder für die Herstellung eines chemischen Modells, wie in Anspruch 7 definiert, umfassend
- eine Verbindung, wie in Anspruch 1 definiert, vorzugsweise Pyridinium-Furosemid, und
- ein Zellsystem, ausgewählt aus einer Zelle, einer Zellkultur, einem Gewebe und einem nichthumanen Tier, das mit der Verbindung in Kontakt gebracht werden soll, um besagtes chemisches Modell zu erhalten.

14. Verfahren gemäß Anspruch 10 oder 12 oder Kit gemäß Anspruch 13, wobei das Zellsystem ein Nematode wie *C. elegans,* ein Zebrafisch oder eine Larve davon ist.

15. Verwendung einer Verbindung, wie in Anspruch 1 definiert, vorzugsweise Pyridinium-Furosemid, zur Induktion bei einem nichthumanen Säuger eines oder mehrerer mit Morbus Parkinson assoziierter Symptome, vorzugsweise ausgewählt aus der Gruppe, bestehend aus Muskelstarre, Ruhetremor, posturaler Instabilität, Akinesie, Schlaf- oder Wachstörung, intrazellulärer Anhäufung von alpha-Synuklein, vorzugsweise in Form von Lewy-Körperchen, Verringerung der mitochondrialen Aktivität, insbesondere des Komplex I, und Degeneration dopaminerger Neuronen der *Substantia nigra*.

16. Verwendung einer Verbindung, wie in Anspruch 1 definiert, vorzugsweise Pyridinium-Furosemid, als Kontrollverbindung oder als Standardverbindung in einem Evaluierungstest der Neurotoxizität einer Verbindung, wobei besagter Evaluierungstest die Inkubation eines Zellsystems, ausgewählt aus einer Zelle, einer Zellkultur, einem Gewebe und einem nichthumanen Tier, mit der Verbindung über einen Zeitraum von mehr als 48 h umfasst.

## Claims

1. Use of a compound of formula (I) 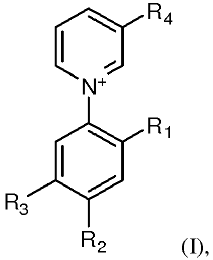 wherein:
- R₁ represents a hydrogen atom, a C₁-C₄ alkyl, a halogen atom, -OH, -CN, -CF₃, -C(=O)NH₂, -C(=O)O⁻, or -C(=O)O-R₅ where R₅ represents H or a C₁-C₄ alkyl,
- R₂ is -SO₂NH₂, a hydrogen atom, a halogen atom, a C₁-C₄ alkyl, -OH, -CN, -C(=O)NH₂, - CF₃, or -C(=O)OR₆ where R₆ represents H or a C₁-C₄ alkyl,
- R₃ is H, a halogen atom, a C₁-C₄ alkyl, -OH, -CN, -C(=O)NH₂,-CF₃, or -C(=O)OR₇ where R₇ represents H or a C₁-C₄ alkyl, and
- R₄ represents a hydrogen atom, -OH, or -OCH₃,
or a pharmaceutically acceptable salt thereof,
in the preparation of a chemical model of a synucleinopathy, preferably Parkinson's disease, by placing a cell system selected from a cell, a cell culture, a tissue, and a non-human animal, in contact with said compound.

2. Method for preparing a chemical model of a synucleinopathy, preferably Parkinson's disease, comprising a step of placing a cell system selected from a cell, a cell culture, a tissue, and a non-human animal, in contact with a compound of formula (I) as defined in claim 1 or an acceptable salt thereof.

3. Use according to claim 1 or method according to claim 2, wherein the chemical model is obtained from nervous tissue, preferably brain tissue, an organotypic culture obtained from brain tissue, a culture of nerve cells, for example a culture of dopaminergic neurons, a primary neuron culture, or a cell line, preferably of neuronal origin.

4. Use according to claim 3 or method according to claim 3, wherein the chemical model is obtained from a cell line selected from the group consisting of the cell lines SH-SY5Y, SK-N-MC, and PC12.

5. Use according to claim 1 or method according to claim 2, wherein the chemical model is a non-human animal, preferably selected from the group consisting of a non-human mammal, an invertebrate such as *C. elegans,* or a fish such as the zebrafish.

6. Use according to claim 1, 3, 4, or 5, or method according to claim 2, 3, 4, or 5, wherein the compound of formula (I) is furosemide pyridinium.

7. Chemical model of a synucleinopathy obtained according to a method as defined in any of claims 2 to 6.

8. Use of a chemical model of a synucleinopathy as defined in claim 7 for the implementation of a screening test for compounds intended for the treatment or prevention of said synucleinopathy, preferably Parkinson's disease.

9. Use of a chemical model of a synucleinopathy as defined in claim 7 for evaluating the effectiveness of a drug substance in the treatment or prevention of said synucleinopathy, said model being a non-human mammal model.

10. Method for evaluating, screening, or selecting a compound intended for the treatment or prevention of a synucleinopathy, preferably Parkinson's disease, said method comprising:
a) placing a cell system selected from a cell, a cell culture, a tissue, and a non-human animal, in contact with a compound as defined in claim 1, preferably furosemide pyridinium,
b) placing said cell system in contact with the test compound, and
c) detecting or quantifying a marker characteristic of the synucleinopathy in order to determine the effectiveness of the test compound,
wherein step b) may be implemented simultaneously, before, or after step a), or may overlap step a).

11. Method according to claim 10, wherein:
- said cell system is selected from the group consisting of nervous tissue, preferably brain tissue, an organotypic culture obtained from brain tissue, a culture of nerve cells, for example neurons that are preferably dopaminergic and/or glial cells, and a cell line preferably of neuronal origin, and
- said marker is selected from the group consisting of an intracellular accumulation of a synuclein, preferably α-synuclein, a marker of apoptotic cell death, preferably an increase of caspase activity, a decrease in the cell survival rate, a decrease in the activity of the mitochondrial respiratory chain, and combinations thereof.

12. Method according to claim 10, wherein:
- said cell system is a non-human animal, and
- said marker characteristic of the synucleinopathy is selected from the group consisting of a motor disorder, particularly a movement disorder or a decrease in rate of movement, a degeneration of dopaminergic neurons, the intracellular accumulation of alpha-synuclein in a dopaminergic neuron, and combinations thereof.

13. Kit for implementing a method for evaluating, screening, or selecting a compound intended for the treatment or prevention of a synucleinopathy, as defined in claim 10, or for the preparation of a chemical model as defined in claim 7, comprising:
- a compound as defined in claim 1, preferably furosemide pyridinium, and
- a cell system selected from a cell, a cell culture, a tissue, and a non-human animal, to be placed in contact with the compound in order to obtain said chemical model.

14. Method according to claim 10 or 12 or kit according to claim 13, wherein the cell system is a nematode such as *C. elegans,* a zebrafish, or a larvae thereof.

15. Use of a compound as defined in claim 1, preferably furosemide pyridinium, to induce in a non-human mammal one or more symptoms associated with Parkinson's disease, preferably selected from the group consisting of muscle rigidity, tremor at rest, postural instability, akinesia, sleep or arousal disorder, intracellular accumulation of alpha-synuclein, preferably in the form of Lewy bodies, a decrease in mitochondrial activity, particularly complex I, and a degeneration of dopaminergic neurons of the *substantia nigra*.

16. Use of a compound as defined in claim 1, preferably furosemide pyridinium, as a control compound or standard compound in a test for evaluating the neurotoxicity of a compound, said test of evaluation comprising incubating a cellular system selected from a cell, a cell culture, a tissue and a non-human animal with the compound for a period greater than 48 hours.
